(19) **Europäisches Patentamt**
**European Patent Office**
**Office européen des brevets**

(11) **EP 2 195 460 B2**

(12) **NEW EUROPEAN PATENT SPECIFICATION**
After opposition procedure

(45) Date of publication and mention
of the opposition decision:
**27.02.2019 Bulletin 2019/09**

(45) Mention of the grant of the patent:
**26.11.2014 Bulletin 2014/48**

(21) Application number: **08806496.9**

(22) Date of filing: **03.10.2008**

(51) Int Cl.:
**C12Q 1/68** (2018.01)    **A23K 1/00** (2006.01)
**A23K 3/00** (2006.01)

(86) International application number:
**PCT/GB2008/003351**

(87) International publication number:
**WO 2009/044152 (09.04.2009 Gazette 2009/15)**

(54) **GENETIC TEST FOR LIVER COPPER ACCUMULATION IN DOGS AND LOW COPPER PET DIET**

GENETISCHER TEST FÜR KUPFERANREICHERUNG IN DER LEBER VON HUNDEN UND
KUPFERARME DIÄT FÜR HAUSTIERE

TEST GÉNÉTIQUE ET RÉGIME D'ANIMAUX DOMESTIQUES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **03.10.2007 GB 0719358**

(43) Date of publication of application:
**16.06.2010 Bulletin 2010/24**

(73) Proprietor: **Mars Incorporated
McLean, VA 22101-3883 (US)**

(72) Inventors:
• **JONES, Paul, Glyn
Melton Mowbray
Leicestershire LE14 4RT (GB)**
• **MARTIN, Alan, James
Melton Mowbray
Leicestershire LE14 4RT (GB)**
• **HOFFMANN, Gaby
NL-3508 TD Utrecht (NL)**
• **ROTHUIZEN, Jan
NL-3508 TD Utrecht (NL)**

(74) Representative: **J A Kemp
14 South Square
Gray's Inn
London WC1R 5JJ (GB)**

(56) References cited:
**EP-A- 1 698 232          WO-A-00/32206
WO-A-99/48384          WO-A-02/056703
WO-A-2004/113570      DE-A1- 19 703 252
US-A1- 2005 123 585   US-B1- 6 911 224**

• **SPEE BART ET AL: "Copper metabolism and
oxidative stress in chronic inflammatory and
cholestatic liver diseases in dogs" JOURNAL OF
VETERINARY INTERNAL MEDICINE, vol. 20, no.
5, September 2006 (2006-09), pages 1085-1092,
XP008103673 ISSN: 0891-6640**
• **SHIH JULIA L ET AL: "Chronic hepatitis in
Labrador Retrievers: Clinical presentation and
prognostic factors" JOURNAL OF VETERINARY
INTERNAL MEDICINE, vol. 21, no. 1, January 2007
(2007-01), pages 33-39, XP008103657 ISSN:
0891-6640**
• **HOFFMANN G ET AL: "Copper-associated
chronic hepatitis in Labrador retrievers"
JOURNAL OF VETERINARY INTERNAL
MEDICINE, vol. 20, no. 4, July 2006 (2006-07),
pages 856-861, XP008103672 ISSN: 0891-6640**
• **HAYWOOD S ET AL: "COPPER TOXICOSIS IN
THE BEDLINGTON TERRIER: A DIAGNOSTIC
DILEMMA" JOURNAL OF SMALL ANIMAL
PRACTICE, BLACKWELL SCIENTIFIC
PUBLICATIONS, OXFORD, GB, vol. 42, no. 4, 1
April 2001 (2001-04-01), pages 181-185,
XP009014397 ISSN: 0022-4510**
• **FRIEDMAN JAMES S ET AL: "Isolation of a
ubiquitin-like (UBL5) gene from a screen
identifying highly expressed and conserved iris
genes" GENOMICS, vol. 71, no. 2, 15 January 2001
(2001-01-15), pages 252-255, XP002519062 ISSN:
0888-7543**
• **SHIMIZU NORIKAZU ET AL: "Treatment and
management of Wilson's disease", PEDIATRICS
INTERNATIONAL, vol. 41, no. 4, August 1999
(1999-08), pages 419-422, ISSN: 1328-8067**
• **MARK MORRIS: 'Small Animal Clinical Nutrition',
vol. 4TH ED., 2000, MARK MORRIS INSTITUTE**

**(Cont. next page)**

Remarks:
   The file contains technical information submitted after
   the application was filed and not included in this
   specification

Remarks:
   The file contains technical information submitted after
   the application was filed and not included in this
   specification

**Description**

**Field of the invention**

The invention relates to a foodstuff for dogs for use in preventing liver copper accumulation and copper-associated liver disease and a method of making the foodstuff.

**Background of the invention**

Although liver diseases are uncommon in dogs, one of its most common forms is chronic hepatitis (CH). CH is a histologic diagnosis, characterised by the presence of fibrosis, inflammation, and hepatocellular apoptosis and necrosis. Cirrhosis can result as the end stage of the disease. One of the causes of CH is hepatic copper accumulation. Hepatic copper accumulation can result from increased uptake of copper, a primary metabolic defect in hepatic copper metabolism, or from altered biliary excretion of copper. Dogs with excessive hepatic copper accumulation are typically treated with D-penicillamine, a potent copper chelator.

**Summary of the invention**

The inventors have discovered that polymorphisms in or in the region of the canine GOLGA5, ATP7a and UBL5 genes are indicative of susceptibility to liver copper accumulation in dogs. The discovery that polymorphisms in these three genomic regions are associated with the risk of liver copper accumulation provides the basis for a test to predict the susceptibility of a dog to liver copper accumulation by screening for the polymorphisms. The predictive power of the test can be magnified using models that involve combining the results of detecting one or more of the defined polymorphisms.

The accumulation of copper in the liver of a dog may lead to one or more diseases or conditions of the liver that are attributable to high liver copper. For example, high liver copper can lead to chronic hepatitis, liver cirrhosis and ultimately liver failure. The test thus enables dogs to be identified which are susceptible to such liver diseases or conditions that are associated with high copper. Once the susceptibility of a dog to liver copper accumulation has been identified it is possible to identify suitable preventative measures for that dog, with the aim of maintaining the liver copper level at a low or normal level, such as by administering the foodstuff of the invention.

Further, and surprisingly, the inventors have now found a foodstuff which is more effective in reducing hepatic copper concentration in Labrador Retrievers than the use of the drug penicillamine. This foodstuff is therefore useful in preventing liver copper accumulation in dogs of the Labrador Retriever breed and can be used for preventing a disease or condition associated with high liver copper such as chronic hepatitis, cirrhosis and liver failure.

Accordingly, the present invention provides a foodstuff comprising copper at a concentration of at least 4.5 to less than 10 mg/kg dry matter for use in preventing a disease attributable to liver copper accumulation in a dog having genetic inheritance of the Labrador Retriever breed, wherein the dog has no clinical symptoms associated with liver copper accumulation.

The present invention further provides:

- use of copper in the manufacture of a foodstuff for a dog having genetic inheritance of the Labrador Retriever breed, wherein the foodstuff comprises copper at a concentration of at least 4.5 to less than 10 mg/kg dry matter and is for use in preventing a disease attributable to liver copper accumulation in said dog, wherein the dog has no clinical symptoms associated with liver copper accumulation;

- a pack comprising a foodstuff having copper at a concentration of at least 4.5 to less than 10 mg/kg dry matter and a zinc supplement for providing a concentration of at least 120 mg/kg dry matter for simultaneous, separate or sequential use in preventing a disease attributable to liver copper accumulation in a dog having genetic inheritance of the Labrador Retriever breed, wherein the dog has no clinical symptoms associated with liver copper accumulation; and

- a labelled foodstuff for use in the method according to the invention or labelled pack for use according to the invention.

**Brief description of the Figures**

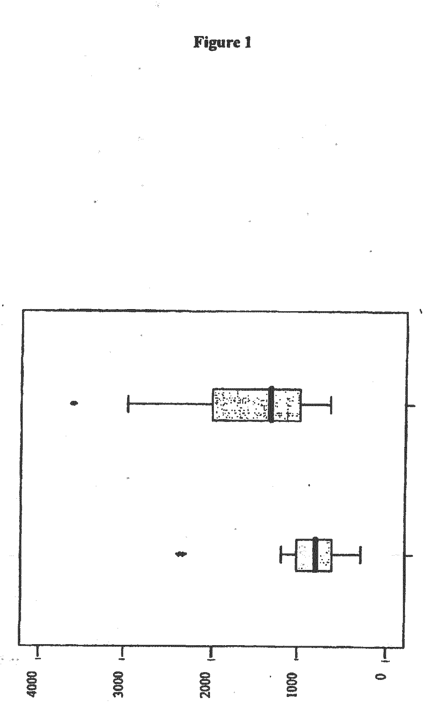

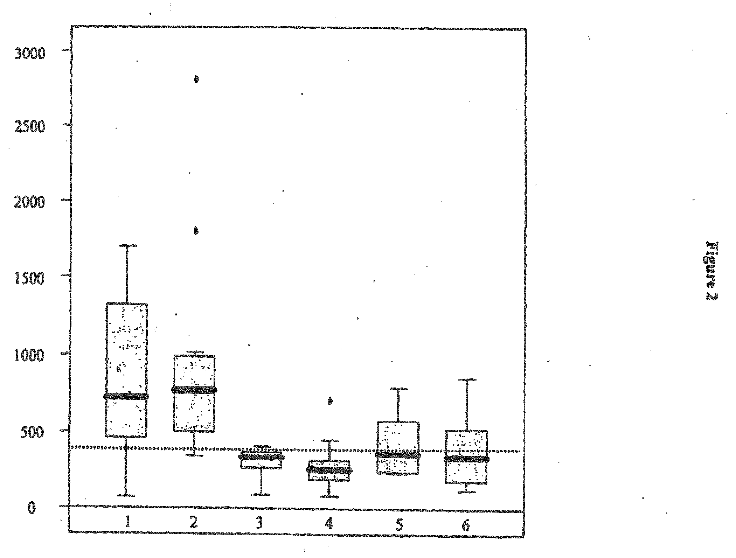

Figure 1 illustrates the progression of hepatic copper accumulation without treatment. The Figure shows hepatic copper concentrations (mg/kg dry weight) of 11 Labrador Retrievers at two examinations 8.7 months (range 6-15 months) apart, prior to any treatment. During this time all animals were fed their usual maintenance diet, which according to the manufacturers contained dietary copper concentrations between 12-25 mg/kg on a dry matter basis and zinc concentrations between 80-270 mg/kg on a dry matter basis. The diamond symbols represent outliers. Figure 2 illustrates hepatic copper concentrations (mg/kg dry weight) of 24 Labrador Retrievers at the beginning of the study and at 2 control examinations during dietary management. The dogs were divided into two groups as follows: group 1 = diet + zinc gluconate tablets, group 2 = diet + placebo. The key to the x-axis numbering is as follows: 1 + 2 = before treatment, 3 + 4 = recheck 1 (first control examination after 8 months (range 5-13 months)), 5 + 6 = recheck 2 (second control examination after 16 months (range 12-25 months)). The numbers of dogs tested are as follows: 1: N = 12 dogs in group 1, 2: N = 12 dogs in group 2, 3: N = 9 dogs in group 1, 4: N = 12 dogs in group 2, 5: N = 6 dogs in group 1, 6: N = 10 dogs in group 2. The diamond symbols represent outliers. The dotted line represents the normal level of hepatic copper for adult dogs.

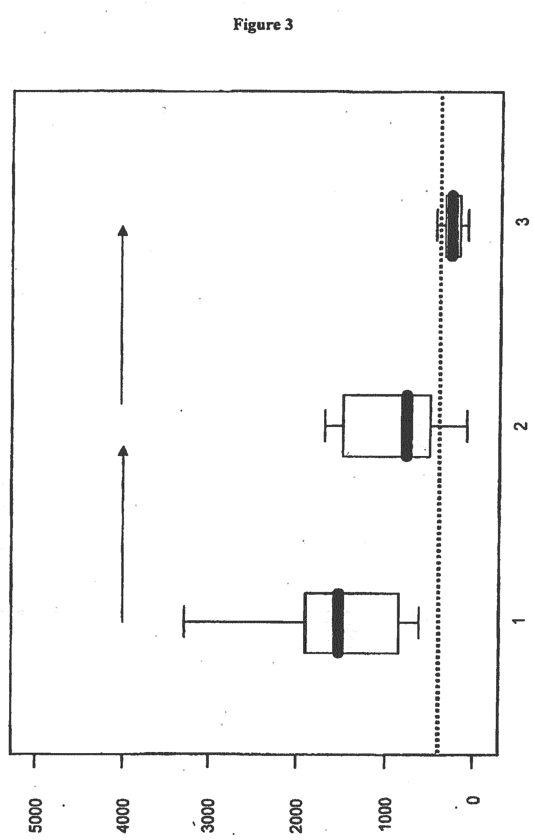

Figure 3 illustrates the effectiveness of the diet of the invention on hepatic copper concentrations (mg/kg dry weight) in 18 Labrador Retrievers compared with the effect of penicillamine alone. The key to the x-axis is as follows: 1 = pre-penicillamine, 2 = post-penicillamine/pre-food, 3 = post-food. The progression along the x-axis from 1 to 2 demonstrates the penicillamine effect, whilst the progression from 2 to 3 demonstrates the food effect. The dotted line represents the normal level of hepatic copper for adult dogs.

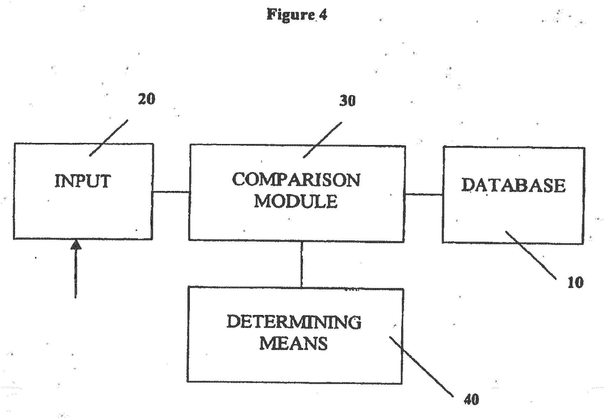

Figure 4 illustrates schematically embodiments of functional components arranged to carry out the present invention.

## Brief description of the sequences

SEQ ID NOs: 1 to 129 show the polynucleotide sequences encompassing the SNPs of the invention.

## Detailed description of the invention

*Identifying susceptibility to liver copper accumulation*

Accumulation of copper in the liver leads to liver disease in a number of dog breeds, including the Labrador Retriever, Doberman Pinscher, German Shepherd, Keeshond, Cocker Spaniel, West Highland White Terrier, Bedlington Terrier, and Skye Terrier. The mean copper concentration in the liver of normal dogs of any breed is 200 to 400 mg/kg on a dry weight basis, although newborns generally have higher liver copper concentrations.

A dog that is susceptible to copper accumulation has a tendency to accumulate copper such that its liver copper concentration reaches a level above 400 mg/kg, for example above 500 mg/kg, 600 mg/kg, 800 mg/kg, 1000 mg/kg, 1500 mg/kg, 2000 mg/kg, 5000 mg/kg, or above 10000 mg/kg. Determining the susceptibility of a dog to liver copper accumulation according to the invention involves determining the risk or likelihood that the dog will accumulate liver copper to a level above 400 mg/kg. This may for example be expressed as a risk factor, percentage or probability. It may be possible to determine whether or not a dog will accumulate copper to the levels described above.

Accumulation of liver copper to a level above 400 mg/kg is associated with liver disease and may ultimately lead to liver failure. Determining the susceptibility of a dog to liver copper accumulation therefore also provides a way of determining the susceptibility of the dog to a disease or condition attributable to liver copper accumulation such as chronic hepatitis, cirrhosis and liver failure.

*Method of elucidating polymorphisms linked to a genetic trait*

The inventors have developed a method of determining the polymorphisms associated with a genetic trait in a group of individuals, namely "Partition Mapping" (also known as "2D mapping"). The method is currently limited to binary conditions (case/control studies).

Complex diseases with a genetic link are generally driven by more than one gene. These genes can interact in non-linear ways, making them more difficult to map using traditional methods. By working on the level of a pair of individuals it is possible to factor out the impact of multiple genes because a locus will either be contributing to the phenotype on that pair of individuals or not. The full working of this process is described below. After running this analysis it is possible to extract the risk alleles in each area and build a model to predict the phenotype using other methods.

The 'Partition Mapping' algorithm scans through the genome stopping every 50 kilobases. At each of these points, every pair of individuals is analysed. For each pair, the genotypes for the whole chromosome are analysed comparing the likelihood of the genotypes under three possible scenarios. The first scenario is that there is a recessive mutation driving the phenotype in this pair of individuals. The second scenario is that there is a dominant mutation driving the phenotype in this pair of individuals. The third scenario is that there is no important mutation for the phenotype in the pair of dogs at this location. The likelihoods are calculated using a hidden markov model, described below. By comparing these likelihoods it is possible to derive a Bayes-Factor for this pair of individuals towards or against the presence of a recessive or dominant phenotype-driving mutation at that point. The log of these values is taken; a positive value then represents more weight towards the recessive or dominant mutation scenario, a negative value represents more evidence towards there being no important mutation here.

The pairs of individuals are sorted in order of the Log-Bayes factors at that locus. The pairs' Log-Bayes-Factors are then summed up in descending order taking a record of the cumulative weight of evidence at each percentile of the data. In most cases some Log-Bayes-Factors will be positive and some will be negative. This will give the effect of the recorded value rising for a percentage of the data and then falling. The maximum of this value gives a measure of the weight of evidence towards either the recessive or dominant models. This is referred to as the 'peak-value'.

In some cases the algorithm has bias towards particularly homozygous areas of the genome or areas with a high density of polymorphisms. This effect is quantified by running the process with every pair permuted across the four possible case/control states (case-case, case-control, control-case, control-control). For any locus one subtracts the peak-value under the permuted model from the normal peak-value, and this gives a corrected peak-value. One can compare the corrected peak-value across the genome giving regions of interest.

*Hidden Markov Model*

A key element of Partition Mapping is that it does not act directly on the genotype data. A layer of analysis that models the degree of relatedness between a pair of dogs is used; this analysis gives information on when two individuals have the same ancestral alleles at a locus in their genome.

There are nine possible relationships between the chromosomes in a pair of dogs, the most related being all four chromosomes of the same haplotype, the least related being four different haplotypes at this point. The likelihoods of these nine states are calculated using a hidden markov model.

Some of these states are impossible under some permutations of the case-control phenotypes if the locus is acting on the pair of individuals in question. Because of this one can calculate Bayes-Factors on the presence of a recessive or dominant allele from the relative likelihoods.

*Polymorphisms and susceptibility to copper accumulation*

Using the method described above, the present inventors have discovered that polymorphisms in or in the region of the canine GOLGA5, ATP7a and UBL5 genes are indicative of susceptibility to liver copper accumulation. Described herein is a method of determining susceptibility of a dog to liver copper accumulation using one or more polymorphic markers at these genomic locations.

The present invention provides a foodstuff for use in a dog determined to be susceptible to liver copper accumulation by a method comprising detecting the presence or absence of (a) a polymorphism in the GOLGA5, ATP7a or UBL5 gene of the dog that is indicative of susceptibility to copper accumulation and/or (b) a polymorphism in linkage disequilibrium with a said polymorphism (a), wherein the dog has no clinical symptoms associated with liver copper accumulation.

The phrase "detecting the presence or absence of a polymorphism" typically means determining whether a polymorphism is present in the genome of the dog. Polymorphisms include Single Nucleotide Polymorphisms (SNP), microsatellite polymorphisms, insertion polymorphisms and deletion polymorphisms. Preferably the polymorphism is a SNP. Detecting the presence or absence of a SNP means genotyping the SNP or typing the nucleotide(s) present in the genome of the dog for the SNP. Typically, the nucleotide present at the same position on both homologous chromosomes will be determined. A dog may therefore be determined to be homozygous for a first allele, heterozygous or homozygous for a second allele of the SNP.

Any one of the polymorphic positions as defined herein may be typed directly, in other words by determining the nucleotide present at that position, or indirectly, for example by determining the nucleotide present at another polymorphic position that is in linkage disequilibrium with said polymorphic position. It would be within the capability of the skilled person to use routine techniques to identify polymorphisms which are in linkage disequilibrium with any one of the polymorphic positions as defined herein.

Polymorphisms which are in linkage disequilibrium with each other in a population are typically found together on the same chromosome. Typically one is found at least 30% of the times, for example at least 40%, at least 50%, at least 70% or at least 90%, of the time the other is found on a particular chromosome in individuals in the population. Thus a polymorphism that is not a functional susceptibility polymorphism, but is in linkage disequilibrium with a functional polymorphism, may act as a marker indicating the presence of the functional polymorphism. A polymorphism that is in linkage disequilibrium with a polymorphism of the invention is indicative of susceptibility to liver copper accumulation.

Polymorphisms which are in linkage disequilibrium with the polymorphisms mentioned herein are typically located within 9mb, preferably within 5mb, within 2mb, within 1mb, within 500kb, within 400kb, within 200kb, within 100kb, within 50kb, within 10kb, within 5kb, within 1kb, within 500bp, within 100bp, within 50bp or within 10bp of the polymorphism.

The disclosure therefore involves detecting the presence or absence of (a) a polymorphism in the GOLGA5, ATP7a or UBL5 gene of the dog that is indicative of susceptibility to copper accumulation and/or (b) a polymorphism in linkage disequilibrium with a said polymorphism (a). Any number and any combination of polymorphisms may be detected to carry out the invention. Preferably at least 2 polymorphisms are detected. Preferably 2 to 5, 3 to 8 or 5 to 10 polymorphisms are detected.

The polymorphism may be present in any one of the GOLGA5, ATP7a or UBL5 genes or may not be present within any one of those genes but is linkage disequilibrium with a polymorphism in any one of those genes. More than one polymorphism may be detected that is in any one of those genes or is in linkage disequilibrium therewith. The polymorphisms may be in any combination.

The DNA of a dog may be typed at the respective positions of:

    (i) two or more polymorphisms (a);
    (ii) two or more polymorphisms (b); or
    (iii) one or more polymorphisms (a) and one or more polymorphisms (b).

When there are two polymorphisms (a), each polymorphism may be in a separate one of the GOLGA5, ATP7a and UBL5 genes or in just one of those genes. When there are three or more polymorphisms (a), for example 3 to 10 such polymorphisms, the polymorphisms may be in the same gene, in two of the genes or in all three genes.

Similarly when there are two polymorphisms (b), each polymorphism may be in linkage disequilibrium with a polymorphism in a separate one of the GOLGA5, ATP7a and UBL5 genes or in just one of those genes. When there are three or more polymorphisms (b), for example 3 to 10 such polymorphisms, the polymorphisms may be in linkage disequilibrium with a polymorphism in the same gene, in two of the genes or in all three genes.

A preferred method comprises detecting the presence or absence of at least one polymorphism (a) in the GOLGA5, ATP7a or UBL5 gene of the dog that is indicative of susceptibility to liver copper accumulation and at least one polymorphism (b) in linkage disequilibrium with a said polymorphism (a).

In a preferred method of the invention, the polymorphism is a SNP. The SNP may be any SNP in the GOLGA5, ATP7a or UBL5 gene of the dog that is indicative of susceptibility to copper accumulation and/or a SNP that is in linkage disequilibrium thereof. Preferably the SNP is selected from a SNP identified in Table III and Table IV. In Tables III and IV each SNP is located at position 61 in the sequence. The wild type and alternative alleles are provided for each SNP at that location. Any number of the SNPs may be used from Tables III and IV and in any combination. The SNPs may be combined with a different type of polymorphism.

Preferably, the method comprises detecting the presence or absence of one or more SNPs selected from the SNPs in Table III and/or one or more SNPs in linkage disequilibrium thereof. Accordingly, any of these 5 SNPs or any SNPs that are in linkage disequilibrium with any of these 5 SNPs may be typed. Preferably at least 2 of these 5 SNPs or SNPs in linkage disequilibrium are typed. More preferably all 5 positions are typed. Preferably therefore, the nucleotide(s) that are typed are selected from positions equivalent to:

- position 61 of SEQ ID NO: 1 (BICF2P506595, SNP1);
- position 61 of SEQ ID NO: 2 (BICF2P772765, SNP 2);
- position 61 of SEQ ID NO: 3 (BICF2S2333187, SNP 3);
- position 61 of SEQ ID NO: 4 (BICF2P1324008, SNP 4);
- position 61 of SEQ ID NO: 5 (BICF2P591872, SNP 5); or any positions which are in linkage disequilibrium with any one of these positions. Preferably, the method comprises detecting the presence or absence of the SNPs BICF2P506595 (SEQ ID NO:1), BICF2P772765 (SEQ ID NO:2), BICF2S2333187 (SEQ ID NO:3), BICF2P1324008 (SEQ ID NO:4), and BICF2P591872 (SEQ ID NO:5).

SNP 1 is located within an intron of the GOLGA5 gene. SNPs 2, 3 and 4 are located in the region of the UBL5 gene. SNP 5 is located in the region of the ATP7a gene. The detection method of the invention therefore relates to any SNP that lies within or in the region of one or more of these genes (in coding regions or otherwise), or any other SNP that is in linkage disequilibrium.

Typing the nucleotide(s) present in the genome of the dog at a position identified in Table III or IV may mean that the nucleotide present at this position in a sequence corresponding exactly with the sequence identified in Table III or IV is typed. However, it will be understood that the exact sequences presented in SEQ ID NOs: 1 to 5 identified in Table III or SEQ ID NO: 6 to 129 in Table IV will not necessarily be present in the dog to be tested. Typing the nucleotide present may therefore be at a position identified in Table III or IV or at an equivalent or corresponding position in the sequence. The term equivalent as used herein therefore means at or at a position corresponding to that identified in Table III or IV. The sequence and thus the position of the SNP could for example vary because of deletions or additions of nucleotides in the genome of the dog. Those skilled in the art will be able to determine a position that corresponds to or is equivalent to the relevant position in each of SEQ ID NOs: 1 to 129, using for example a computer program such as GAP, BESTFIT, COMPARE, ALIGN, PILEUP or BLAST. The UWGCG Package provides programs including GAP, BESTFIT, COMPARE, ALIGN and PILEUP that can be used to calculate homology or line up sequences (for example used on their default settings). The BLAST algorithm can also be used to compare or line up two sequences, typically on its default settings. Software for performing a BLAST comparison of two sequences is publicly available through the National Center for Biotechnology Information (http://www.ncbi.nlm.nih.gov/). This algorithm is further described below. Similar publicly available tools for the alignment and comparison of sequences may be found on the European Bioinformatics Institute website (http://www.ebi.ac.uk), for example the ALIGN and CLUSTALW programs.

Once the presence or absence of the one or more polymorphisms of the invention have been detected in the genome of the dog, the susceptibility of the dog to liver copper accumulation is thereby determined. The genotype of each polymorphism alone or in combination with other polymorphisms is indicative of the susceptibility of the dog to liver copper accumulation. As an example, Table I sets out the different possible genotypes of the combination of 5 SNPs in the region of the GOLGA5, UBL5 and ATP7a genes and the percentage of dogs with those genotypes that have high copper (liver levels of above 600 mg/kg). In this example, to determine the susceptibility of a dog to liver copper accumulation one may genotype the 5 SNPs in the genome of the dog using a DNA sample from the dog. Once the genotypes of the SNPs have been determined, these can be converted into binary values based on the key provided in Example 1, i.e. based on the degree of association of the genotype with high copper. Then, Table I is used to convert the binary values into a risk factor based on the percentage of dogs that have that genotype pattern and high copper.

A dog may be tested by a method of the invention at any age, for example from 0 to 12,0 to 6,0 to 5, 0 to 4,0 to 3, 0 to 2 or 0 to 1 years old. Preferably the dog is tested at as young an age as possible, for example within the first year, first 6 months or first 3 months of its life. The dog is preferably tested before copper accumulation occurs. The history of the dog may or may not be known. For example, the dog may be a pup of known parents and the history of the parents with respect to copper accumulation may be known. Alternatively, the dog may be a stray or a rescued dog with unknown parentage and history.

The dog has genetic breed inheritance of Labrador Retriever. The dog may be a purebred Labrador Retriever. Alternatively, the dog may be a mixed or crossbred dog, or an outbred dog (mongrel). One or both of the parents of the dog may be a pure-bred Labrador Retriever dog. One, two, three or four of the grandparents of the dog may be a pure-bred Labrador Retriever dog. The dog may have at least 50% or at least 75% of the Labrador Retriever breed in its genetic background. Thus, at least 50% or at least 75% of the dog's genome may be derived from the Labrador Retriever breed.

The genetic breed background of a dog may be determined by assessing the allelic frequencies of genetic markers, for example SNPs or microsatellites. The combinations of allelic frequencies of different SNPs or microsatellites in a dog provide a signature that allows the breed of a dog or the breeds that make up a mixed breed dog to be determined. Such a genetic test may be a commercially available test. Alternatively, the dog may not need to be tested for Labrador Retriever breed

inheritance because it is suspected of having a Labrador Retriever breed inheritance for example by the dog owner or veterinarian. This could be for example because of knowledge of the dog's ancestry or because of its appearance.

The predictive test of the invention may be carried out in conjunction with one or more other predictive or diagnostic tests such as determining the genetic breed background of the dog or susceptibility to one or more other diseases.

*Detection of polymorphisms*

The detection of polymorphisms according to the invention may comprise contacting a polynucleotide or protein of the dog with a specific binding agent for a polymorphism and determining whether the agent binds to the polynucleotide or protein, wherein binding of the agent indicates the presence of the polymorphism, and lack of binding of the agent indicates the absence of the polymorphism.

The method is generally carried *out in vitro* on a sample from the dog, where the sample contains DNA from the dog. The sample typically comprises a body fluid and/or cells of the dog and may, for example, be obtained using a swab, such as a mouth swab. The sample may be a blood, urine, saliva, skin, cheek cell or hair root sample. The sample is typically processed before the method is carried out, for example DNA extraction may be carried out. The polynucleotide or protein in the sample may be cleaved either physically or chemically, for example using a suitable enzyme. In one embodiment the part of polynucleotide in the sample is copied or amplified, for example by cloning or using a PCR based method prior to detecting the polymorphism.

In the present invention, any one or more methods may comprise determining the presence or absence of one or more polymorphisms in the dog. The polymorphism is typically detected by directly determining the presence of the polymorphic sequence in a polynucleotide or protein of the dog. Such a polynucleotide is typically genomic DNA, mRNA or cDNA. The polymorphism may be detected by any suitable method such as those mentioned below.

A specific binding agent is an agent that binds with preferential or high affinity to the protein or polypeptide having the polymorphism but does not bind or binds with only low affinity to other polypeptides or proteins. The specific binding agent may be a probe or primer. The probe may be a protein (such as an antibody) or an oligonucleotide. The probe may be labelled or may be capable of being labelled indirectly. The binding of the probe to the polynucleotide or protein may be used to immobilise either the probe or the polynucleotide or protein.

Generally in the method, a polymorphism can be detected by determining the binding of the agent to the polymorphic polynucleotide or protein of the dog. However in one embodiment the agent is also able to bind the corresponding wild-type sequence, for example by binding the nucleotides or amino acids which flank the variant position, although the manner of binding to the wild-type sequence will be detectably different to the binding of a polynucleotide or protein containing the polymorphism.

The method may be based on an oligonucleotide ligation assay in which two oligonucleotide probes are used. These probes bind to adjacent areas on the polynucleotide that contains the polymorphism, allowing after binding the two probes to be ligated together by an appropriate ligase enzyme. However the presence of a single mismatch within one of the probes may disrupt binding and ligation. Thus ligated probes will only occur with a polynucleotide that contains the polymorphism, and therefore the detection of the ligated product may be used to determine the presence of the polymorphism.

In one embodiment the probe is used in a heteroduplex analysis based system. In such a system when the probe is bound to a polynucleotide sequence containing the polymorphism it forms a heteroduplex at the site where the polymorphism occurs and hence does not form a double strand structure. Such a heteroduplex structure can be detected by the use of a single or double strand specific enzyme. Typically the probe is an RNA probe, the heteroduplex region is cleaved using RNAase H and the polymorphism is detected by detecting the cleavage products.

The method may be based on fluorescent chemical cleavage mismatch analysis which is described for example in PCR Methods and Applications 3, 268-71 (1994) and Proc. Natl. Acad. Sci. 85,4397-4401 (1998).

In one embodiment a PCR primer is used that primes a PCR reaction only if it binds a polynucleotide containing the polymorphism, for example a sequence-specific PCR system, and the presence of the polymorphism may be determined by detecting the PCR product. Preferably the region of the primer that is complementary to the polymorphism is at or near the 3' end of the primer. The presence of the polymorphism may be determined using a fluorescent dye and quenching agent-based PCR assay such as the Taqman PCR detection system.

The specific binding agent may be capable of specifically binding the amino acid sequence encoded by a polymorphic sequence. For example, the agent may be an antibody or antibody fragment. The detection method may be based on an ELISA system. The method may be an RFLP based system. This can be used if the presence of the polymorphism in the polynucleotide creates or destroys a restriction site that is recognised by a restriction enzyme.

The presence of the polymorphism may be determined based on the change that the presence of the polymorphism makes to the mobility of the polynucleotide or protein during gel electrophoresis. In the case of a polynucleotide, single-stranded conformation polymorphism (SSCP) or denaturing gradient gel electrophoresis (DDGE) analysis may be used. In another method of detecting the polymorphism, a polynucleotide comprising the polymorphic region is sequenced across the region that contains the polymorphism to determine the presence of the polymorphism.

The presence of the polymorphism may be detected by means of fluorescence resonance energy transfer (FRET). In particular, the polymorphism may be detected by means of a dual hybridisation probe system. This method involves the use of two oligonucleotide probes that are located close to each other and that are complementary to an internal segment of a target polynucleotide of interest, where each of the two probes is labelled with a fluorophore. Any suitable fluorescent label or dye may be used as the fluorophore, such that the emission wavelength of the fluorophore on one probe (the donor) overlaps the excitation wavelength of the fluorophore on the second probe (the acceptor). A typical donor fluorophore is fluorescein (FAM), and typical acceptor fluorophores include Texas red, rhodamine, LC-640, LC-705 and cyanine 5 (Cy5).

In order for fluorescence resonance energy transfer to take place, the two fluorophores need to come into close proximity on hybridisation of both probes to the target. When the donor fluorophore is excited with an appropriate wavelength of light, the emission spectrum energy is transferred to the fluorophore on the acceptor probe resulting in its fluorescence. Therefore, detection of this wavelength of light, during excitation at the wavelength appropriate for the donor fluorophore, indicates hybridisation and close association of the fluorophores on the two probes. Each probe may be labelled with a fluorophore at one end such that the probe located upstream (5') is labelled at its 3' end, and the probe located downstream (3') is labelled at its 5' end. The gap between the two probes when bound to the target sequence may be from 1 to 20 nucleotides, preferably from 1 to 17 nucleotides, more preferably from 1 to 10 nucleotides, such as a gap of 1, 2, 4, 6, 8 or 10 nucleotides.

The first of the two probes may be designed to bind to a conserved sequence of the gene adjacent to a polymorphism and the second probe may be designed to bind to a region including one or more polymorphisms. Polymorphisms within the sequence of the gene targeted by the second probe can be detected by measuring the change in melting temperature caused by the resulting base mismatches. The extent of the change in the melting temperature will be dependent on the number and base types involved in the nucleotide polymorphisms.

Polymorphism typing may also be performed using a primer extension technique. In this technique, the target region surrounding the polymorphic site is copied or amplified for example using PCR. A single base sequencing reaction is then performed using a primer that anneals one base away from the polymorphic site (allele-specific nucleotide incorporation). The primer extension product is then detected to determine the nucleotide present at the polymorphic site. There are several ways in which the extension product can be detected. In one detection method for example, fluorescently labelled dideoxynucleotide terminators are used to stop the extension reaction at the polymorphic site. Alternatively, mass-modified dideoxynucleotide terminators are used and the primer extension products are detected using mass spectrometry. By specifically labelling one or more of the terminators, the sequence of the extended primer, and hence the nucleotide present at the polymorphic site can be deduced. More than one reaction product can be analysed per reaction and consequently the nucleotide present on both homologous chromosomes can be determined if more than one terminator is specifically labelled.

We describe primers or probes that may be used in the detection of any of the SNPs defined herein for use in the prediction of susceptibility to copper accumulation. Polynucleotides of the invention may also be used as primers for primer extension reactions to detect the SNPs defined herein.

Such primers, probes and other polynucleotide fragments will preferably be at least 10, preferably at least 15 or at least 20, for example at least 25, at least 30 or at least 40 nucleotides in length. They will typically be up to 40, 50, 60, 70, 100 or 150 nucleotides in length. Probes and fragments can be longer than 150 nucleotides in length, for example up to 200, 300, 400, 500, 600, 700 nucleotides in length, or even up to a few nucleotides, such as five or ten nucleotides, short of a full length polynucleotide sequence of the invention.

Primers and probes for genotyping the SNPs of the invention may be designed using any suitable design software known in the art using the SNP sequences in Tables III and IV. Homologues of these polynucleotide sequences would also be suitable for designing primers and probes. Such homologues typically have at least 70% homology, preferably at least 80, 90%, 95%, 97% or 99% homology, for example over a region of at least 15, 20, 30, 100 more contiguous nucleotide. The homology may be calculated on the basis of nucleotide identity (sometimes referred to as "hard homology").

For example the UWGCG Package provides the BESTFIT program that can be used to calculate homology (for example used on its default settings) (Devereux et al (1984) Nucleic Acids Research 12, p387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as default a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad Sci. USA 90: 5873-5787. One measure of similarity provided by the BLAST algorithm is the smallest sum probability (P(N)), which provides an indication of the probability by which a match between two polynucleotide sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by at least 1,2,5,10, 20 or more mutations, which may be substitutions, deletions or insertions of nucleotides

The polynucleotides described herein such as primers or probes may be present in an isolated or substantially purified form. They may be mixed with carriers or diluents that will not interfere with their intended use and still be regarded as substantially isolated. They may also be in a substantially purified form, in which case they will generally comprise at least 90%, e.g. at least 95%, 98% or 99%, of polynucleotides of the preparation.

*Detector antibodies*

A detector antibody is an antibody that is specific for one polymorphism but does not bind to any other polymorphism as described herein. Detector antibodies are for example useful in purification, isolation or screening methods involving immunoprecipitation techniques.

Antibodies may be raised against specific epitopes of the polypeptides of the invention. An antibody, or other compound, "specifically binds" to a polypeptide when it binds with preferential or high affinity to the protein for which it is specific but does substantially bind not bind or binds with only low affinity to other polypeptides. A variety of protocols for competitive binding or immunoradiometric assays to determine the specific binding capability of an antibody are well known in the art (see for example Maddox et al, J. Exp. Med. 158, 1211-1226, 1993). Such immunoassays typically involve the formation of complexes between the specific protein and its antibody and the measurement of complex formation.

For the purposes of this invention, the term "antibody", unless specified to the contrary, includes fragments that bind a polypeptide of the invention. Such fragments include Fv, F(ab') and F(ab')$_2$ fragments, as well as single chain antibodies. Furthermore, the antibodies and fragment thereof may be chimeric antibodies, CDR-grafted antibodies or humanised antibodies.

Antibodies may be used in a method for detecting polypeptides of the invention in a biological sample (such as any such sample mentioned herein), which method comprises:

I providing an antibody of the invention;
II incubating a biological sample with said antibody under conditions which allow for the formation of an antibody-antigen complex; and
III determining whether antibody-antigen complex comprising said antibody is formed.

Antibodies of the invention can be produced by any suitable method. Means for preparing and characterising antibodies are well known in the art, see for example Harlow and Lane (1988) "Antibodies: A Laboratory Manual", Cold Spring Harbor Laboratory Press, Cold Spring Harbor, NY. For example, an antibody may be produced by raising an antibody in a host animal against the whole polypeptide or a fragment thereof, for example an antigenic epitope thereof, hereinafter the "immunogen". The fragment may be any of the fragments mentioned herein (typically at least 10 or at least 15 amino acids long).

A method for producing a polyclonal antibody comprises immunising a suitable host animal, for example an experimental animal, with the immunogen and isolating immunoglobulins from the animal's serum. The animal may therefore be inoculated with the immunogen, blood subsequently removed from the animal and the IgG fraction purified. A method for producing a monoclonal antibody comprises immortalising cells which produce the desired antibody. Hybridoma cells may be produced by fusing spleen cells from an inoculated experimental animal with tumour cells (Kohler and Milstein (1975) Nature 256, 495-497).

An immortalized cell producing the desired antibody may be selected by a conventional procedure. The hybridomas may be grown in culture or injected intraperitoneally for formation of ascites fluid or into the blood stream of an allogenic host or immunocompromised host. Human antibody may be prepared *by in vitro* immunisation of human lymphocytes, followed by transformation of the lymphocytes with Epstein-Barr virus.

For the production of both monoclonal and polyclonal antibodies, the experimental animal is suitably a goat, rabbit, rat, mouse, guinea pig, chicken, sheep or horse. If desired, the immunogen may be administered as a conjugate in which the immunogen is coupled, for example via a side chain of one of the amino acid residues, to a suitable carrier. The carrier molecule is typically a physiologically acceptable carrier. The antibody obtained may be isolated and, if desired, purified.

*Detection kit*

We also describe a kit that comprises means for typing one or more of the polymorphisms defined herein. In particular, such means may include a specific binding agent, probe, primer, pair or combination of primers, or antibody, including an antibody fragment, as defined herein which is capable of detecting or aiding detection of the polymorphisms defined herein. The primer or pair or combination of primers may be sequence specific primers that only cause PCR amplification of a polynucleotide sequence comprising the polymorphism to be detected, as discussed herein. The primer or pair of primers may alternatively not be specific for the polymorphic nucleotide, but may be specific for the region upstream (5') and/or downstream (3'). These primers allow the region encompassing the polymorphic nucleotide to be copied. A kit suitable for use in the primer-extension technique may specifically include labelled dideoxynucleotide triphosphates (ddNTPs). These may for example be fluorescently labelled or mass modified to enable detection of the extension product and consequently determination of the nucleotide present at the polymorphic position.

The kit may also comprise a specific binding agent, probe, primer, pair or combination of primers, or antibody that is capable of detecting the absence of the polymorphism. The kit may further comprise buffers or aqueous solutions.

The kit may additionally comprise one or more other reagents or instruments that enable any of the embodiments of the method mentioned above to be carried out. Such reagents or instruments may include one or more of the following: a means to detect the binding of the agent to the polymorphism, a detectable label such as a fluorescent label, an enzyme able to act on a polynucleotide, typically a polymerase, restriction enzyme, ligase, RNAse H or an enzyme which can attach a label to a polynucleotide, suitable buffer(s) or aqueous solutions for enzyme reagents, PCR primers which bind to regions flanking the polymorphism as discussed herein, a positive and/or negative control, a gel electrophoresis apparatus, a means to isolate DNA from sample, a means to obtain a sample from the individual, such as swab or an instrument comprising a needle, or a support comprising wells on which detection reactions can be carried out. The kit may be, or include, an array such as a polynucleotide array comprising the specific binding agent, preferably a probe, of the invention. The kit typically includes a set of instructions for using the kit.

*Bioinformatics*

The sequences of the polymorphisms may be stored in an electronic format, for example in a computer database. Accordingly, we describe a database comprising information relating to one or more polymorphisms in the GOLGA5, ATP7a or UBL5 genes and/or one or more polymorphisms in linkage disequilibrium thereof and their association with the susceptibility of a dog to liver copper accumulation. The database may include further information about the polymorphism, for example the degree of association of the polymorphism with the susceptibility to liver copper accumulation.

A database as described herein may be used to predict the susceptibility of a dog to liver copper accumulation. Such a determination may be carried out by electronic means, for example by using a computer system (such as a PC). Typically, the determination will be carried out by inputting to a computer system genetic data from the dog to a computer system; comparing the genetic data to a database comprising information relating to one or more polymorphisms in the GOLGA5, ATP7a or UBL5 genes and/or one or more polymorphisms in linkage disequilibrium thereof and their association with the susceptibility of a dog to liver copper accumulation; and on the basis of this comparison, predicting the susceptibility of the dog to liver copper accumulation. This information can then be used to guide the management of the liver copper levels of the dog.

Also described is a computer program comprising program code means for performing all the steps of a method described herein when said program is run on a computer. Also described is a computer program product comprising program code means stored on a computer readable medium for performing a method described herein when said program is run on a computer. A computer program product comprising program code means on a carrier wave that, when executed on a computer system, instruct the computer system to perform a method described herein is additionally described.

As illustrated in Figure 4, an apparatus can be arranged to perform a method described herein. The apparatus typically comprises a computer system, such as a PC. The computer system may comprise: means 20 for receiving genetic data from the dog; a module 30 for comparing the data with a database 10 comprising information relating to polymorphisms; and means 40 for predicting on the basis of said comparison the susceptibility of the dog to liver copper accumulation.

*Foodstuff of the invention*

The present invention is concerned with a foodstuff for dogs having the genetic inheritance of the Labrador Retriever breed. The inventors found that the level of copper found in commercial diets is associated with liver copper accumulation in Labrador Retrievers and that reducing the level of copper in the diet surprisingly allows the liver copper level to be brought to a normal level more efficiently than the drug penicillamine. The foodstuff of the invention has a low copper concentration, specifically a copper concentration of at least 4.5 to less than 10 mg/kg dry matter. The foodstuff is used to prevent the accumulation of copper in the livers of Labrador Retrievers, wherein the dogs have no clinical symptoms associated with liver copper accumulation. It is therefore useful for preventing a disease or condition attributable to liver copper accumulation. The term "foodstuff" as used herein covers foodstuff, diet, comestible or supplement. Any of these forms may be solid, semi-solid or liquid.

In more detail, the foodstuff of the invention comprises copper at a concentration of at least 4.5 to less than 10 mg/kg dry matter. Preferably, the copper concentration is at least 4.75, at least 5 or at least 8 mg/kg dry matter. The copper may be present in the foodstuff in any physiologically acceptable form, for example as copper sulphate.

The foodstuff may further comprise zinc at a concentration of at least 120 mg/kg dry matter. Preferably the zinc concentration is at least 150, at least 180 or at least 200 mg/kg dry matter. Preferably the zinc concentration does not exceed the maximum allowed by food regulatory authorities. Typically, the zinc concentration is less than 250, less than 240, less than 230 or less than 220 mg/kg dry matter. Typically, the zinc concentration will be in the range of 120 to 250,150 to 250 or 200 to 250 mg/kg dry matter.

The zinc may be provided in the same foodstuff providing the level of copper of the invention. Alternatively, zinc may be provided in the form of a supplement, which can be added to a foodstuff of the invention. A supplement can be in the form of a tablet, powder or liquid formulation. Zinc may be provided in the form of any physiologically acceptable salt. Examples of zinc sources include zinc acetate, zinc sulphate, zinc gluconate, zinc carbonate, zinc chloride and zinc oxide.

The concentration of copper or zinc in the foodstuff is described herein on a dry matter basis, i.e. on the basis of the foodstuff without water, to enable direct comparison between different foodstuffs that may have different moisture content To measure the concentration of copper or zinc in a wet or semi-wet foodstuff, a sample of the foodstuff is first dried, for example using an oven, to remove water. Thereafter, any suitable technique may be used to measure the concentration of copper or zinc in the sample of the dried foodstuff. An example of such a technique that is well known in the art is flame atomic absorption spectrophotometry.

The foodstuff of the invention may be in the form of, for example, a wet pet food, a semi-moist pet food or a dry pet food. Wet pet food generally has a moisture content above 65% by weight. Semi-moist pet food typically has a moisture content between 20-65% by weight and can include humectants and other ingredients to prevent microbial growth. Dry pet food, also called kibble, generally has a moisture content below 20% by weight and its processing typically includes extruding, drying and/or baking in heat.

The foodstuff may be provided as a mixture of wet and dry food. Such a combination may be provided premixed or may by provided as two or more separate foodstuffs, which are provided to the dog separately, simultaneously or sequentially.

The foodstuff encompasses any product that the dog consumes in its diet. The invention covers standard food products as well as pet food snacks. The foodstuff is preferably a cooked product. It may incorporate meat or animal derived material (such as beef, chicken, turkey, lamb, fish etc). The product may alternatively be meat-free (preferably including a meat substitute such as soya, maize gluten or a soya product in order to provide a protein source). The product may also contain a starch source such as one or more grains (e.g. com, rice, oats, barley etc), or may be starch free.

The ingredients of a dry pet food may be selected from cereal, grain, meat, poultry, fat, vitamin and mineral. The ingredients are typically mixed and put through an extruder/cooker. The product is then typically shaped and dried, and after drying, flavours and fats may be coated or sprayed onto the dry product.

All pet food is required to provide a certain level of nutrients. For example, the Association of American Feed Control Officials (AAFCO) and the Pet Food Institute have established nutrient profiles for dog foods, based on commonly used ingredients. These established profiles are called the "AAFCO dog food nutrient profiles". Under these regulations, dog foods must be formulated to contain concentrations of nutrients that meet all minimum levels and not to exceed the maximum levels as determined by AAFCO.

The dog food formulation may be customised according to the caloric, protein, fat, carbohydrate, fibre, vitamin or mineral requirements of the dog. For example, the dog food formulation may be customised to provide the correct amounts or ratio of essential fatty acids such as omega-6 and omega-3 fatty acids. The main sources of omega-6 fatty acids are plants such as sunflower, soyabean oil, safflower and evening primrose oil, whereas omega-3 fatty acids are mainly found in linseed and marine sources. Food ingredients that are high in copper and may be avoided in the production of the foodstuff include shellfish, liver, kidney, heart, meat, nuts, mushrooms, cereals, cocoa, legumes and soft water (copper pipes). Food ingredients that are rich in zinc and may be included in the formulation include milk, gelatin, egg yolks, rice and potatoes.

The foodstuff is preferably packaged. The packaging may be metal (usually in the form of a tin or flexifoil), plastic (usually in the form of a pouch or bottle), paper or card. The amount of moisture in any product may influence the type of packaging, which can be used or is required.

The foodstuff of the invention may be packaged together with a source of zinc. Zinc may be provided in any form. Zinc may be provided within one or more foodstuffs or in the form of a separate supplement that is packaged with the foodstuff comprising the maximum level of copper of the invention. When the zinc is provided in a foodstuff, it may be provided within the same foodstuff containing the particular level of copper of the invention or it may be provided in one or more separate foodstuffs or both. The invention therefore provides a pack comprising a foodstuff having copper at a concentration of at least 4.5 to less than 10 mg/kg dry matter and a zinc supplement. The zinc supplement provides a concentration of at least 120 mg/kg dry matter when added to the foodstuff. The foodstuff and zinc supplement are for simultaneous, separate or sequential use in preventing a disease attributable to liver copper accumulation in a dog having genetic inheritance of the Labrador Retriever breed, wherein the dog has no clinical symptoms associated with liver copper accumulation.

The invention also provides a labelled foodstuff as discussed herein. The label may for example indicate that the foodstuff is suitable for a dog of the Labrador Retriever breed. Other indications or instructions could be provided. For example, the amount of copper and/or zinc that the diet or foodstuff contains, in addition to other ingredients, may be stated. Furthermore, feeding instructions could be provided.

*Labrador Retriever*

The foodstuff of the invention is suitable for preventing liver copper accumulation in a dog having genetic inheritance of the Labrador Retriever breed, wherein the dog has no clinical symptoms associated with liver copper accumulation. The dog is typically a companion dog or pet. The dog may be a purebred Labrador Retriever. Alternatively, the dog may be a mixed or crossbred dog, or an outbred dog (mongrel). One or both of the parents of the dog may be a pure-bred Labrador Retriever dog. One, two, three or four of the grandparents of the dog may be a pure-bred Labrador Retriever dog. The dog may have at least 50% or at least 75% of the Labrador Retriever breed in its genetic background. Thus, at least 50% or at least 75% of the dog's genome may be derived from the Labrador Retriever breed.

The genetic breed background of a dog may be determined by assessing the allelic frequencies of genetic markers, for example SNPs or microsatellites. The combinations of allelic frequencies of different SNPs or microsatellites in a dog provide a signature that allows the breed of a dog or the breeds that make up a mixed breed dog to be determined. Such a genetic test may be a commercially available test. Alternatively, the dog may not need to be tested for Labrador Retriever breed inheritance because it is suspected of having a Labrador Retriever breed inheritance for example by the dog owner or veterinarian. This could be for example because of knowledge of the dog's ancestry or because of its appearance.

The food is suitable for a dog of any age. The food may be suitable for a dog that has an age of from 0 to 12 years old, for example from 1 to 5 years old, from 2 to 7 years old or from 3 to 9 years old.

Generally, the foodstuff is suitable for a healthy dog. It is suitable for a dog that does not have a detectable accumulation of hepatic copper. The foodstuff is intended for prophylactic use, i.e. to prevent the accumulation of copper in the liver of a dog. The foodstuff is intended for minimising the risk of copper accumulation and thereby reducing the probability of the dog from developing a disease or condition attributable to liver copper accumulation such as chronic hepatitis, cirrhosis or liver failure. Typically, the foodstuff is for use in preventing copper accumulation in a dog that does not have an abnormal hepatic copper concentration and is therefore not likely to be at risk of suffering from copper-associated hepatitis. The dog may also have no history of accumulating copper. The dog therefore preferably has a normal level of hepatic copper in the range of less than about 400 mg/kg of dry liver weight. However, in newborn dogs the normal level of hepatic copper may be considered to be less than about 600 mg/kg dry liver weight. The aim of providing the foodstuff to the dog is to prevent the hepatic copper concentration from reaching levels significantly higher than the normal level. Methods that can be used to determine the concentration of copper in the liver of a dog are well known in the art. A suitable method is described in Example 1.

The foodstuff can be used for preventing copper-associated chronic hepatitis. The foodstuff is preferably for use in preventing copper accumulation in a dog that does not have detectable liver disease, with the aim of preventing such liver disease. The foodstuff could also be used to treat a disease or condition attributable to liver copper accumulation, such as chronic hepatitis, cirrhosis or liver failure. Evidence or symptoms of liver disease include clinical indications such as lethargy, diarrhoea and icterus. Biochemical indications of liver disease include abnormally increased serum bilirubin and serum liver enzyme activities such as alkaline phosphatase (ALP), alanine aminotransferase (ALT), aspartate aminotransferase (AST) and gamma-glutamyl transpeptidase. The biochemical measurement of such liver disease indicators is well known in the art.

Preferably the dog that the food is intended for does not have any clinical problems.

*Food manufacturing*

The foodstuff of the invention can be made by mixing together suitable ingredients. The manufacture is controlled so that the foodstuff has the required copper concentration. The concentration of copper may be monitored during the foodstuff production process. The foodstuff of the invention may be made by mixing together the ingredients for the foodstuff so that the foodstuff has a copper concentration of at least 4.5 to less than 10 mg/kg dry matter. One or more of the components to be incorporated into the foodstuff may provide a source of copper. However it is important to avoid the use of components that are likely to be rich in copper. Food ingredients that are high in copper and may be avoided in the production of the foodstuff include shellfish, liver, kidney, heart, meat, nuts, mushrooms, cereals, cocoa, legumes and soft water (copper pipes). Optionally, one or more of the components will provide a source of zinc for the foodstuff of invention. Food ingredients that are rich in zinc and may be included in the formulation include milk, gelatin, egg yolks, rice and potatoes. The components/ingredients may be added at any time during the manufacture/processing of the foodstuff.

It is important to measure the concentration of copper that is present in the foodstuff to determine that the concentration is below the limit according to the invention. The concentration of zinc could also be measured to check that it is above the minimum level that is preferred according to the invention. One of the steps in the method of manufacture of the foodstuff may comprise measuring the copper concentration in a sample of the foodstuff. At least one measurement of the copper concentration may be made from a sample of the foodstuff after the foodstuff has been prepared. Measurements could also be made during the preparation of the foodstuff in order to monitor the levels of copper and/or zinc that are accumulating by the addition of further ingredients. For example, a measurement could be made on a sample after the addition of one or more ingredients. Measurements of copper, zinc and other elements can be made on a sample using any suitable method known in the art such as flame atomic absorption spectrophotometry.

Typically, the method of making the foodstuff comprises the steps of mixing together the ingredients with optional cooking of any raw ingredients; measuring the concentration of copper, and optionally zinc, in a sample of the foodstuff; and packaging the foodstuff. The method of making the foodstuff may further comprise providing the dog's owner, the person responsible for feeding the dog or a vet with the foodstuff and/or providing the foodstuff to the dog.

Whilst it would be unusual for a foodstuff not to contain any copper, copper could be added as a supplement to the foodstuff in order to achieve the minimum daily requirement of copper in the dog's diet (for example as recommended by the American Feed Control Official (AAFCO)) whilst still maintaining the copper concentration at a level below that required by the invention. The invention therefore also provides the use of copper in the manufacture of a foodstuff for a dog having genetic inheritance of the Labrador Retriever breed, wherein the foodstuff comprises copper at a concentration of at least 4.5 to less than 10 mg/kg and is for use in preventing copper accumulation in said dog, wherein the dog has no clinical symptoms associated with liver copper accumulation. The copper may be added to the foodstuff in any suitable form. Examples of copper supplements include cupric chloride and cupric sulphate pentahydrate.

The food product manufacturing apparatus used in the present invention typically comprises one or more of the following components: container for dry pet food ingredients; container for liquids; mixer; former and/or extruder; cut-off device; cooking means (e.g. oven); cooler; packaging means; and labelling means. A dry ingredient container typically has an opening at the bottom. This opening may be covered by a volume-regulating element, such as a rotary lock. The volume-regulating element may be opened and closed according to the electronic manufacturing instructions to regulate the addition of dry ingredients to the pet food. Dry ingredients typically used in the manufacture of pet food include corn, wheat, meat and/or poultry meal. Liquid ingredients typically used in the manufacture of pet food include fat, tallow and water. A liquid container may contain a pump that can be controlled, for example by the electronic manufacturing instructions, to add a measured amount of liquid to the pet food.

The dry ingredient container(s) and the liquid container(s) may be coupled to a mixer and deliver the specified amounts of dry ingredients and liquids to the mixer. The mixer may be controlled by the electronic manufacturing instructions. For example, the duration or speed of mixing may be controlled. The mixed ingredients are typically then delivered to a former or extruder. The former/extruder may be any former or extruder known in the art that can be used to shape the mixed ingredients into the required shape. Typically, the mixed ingredients are forced through a restricted opening under pressure to form a continuous strand. As the strand is extruded, it may be cut into pieces (kibbles) by a cut-off device, such as a knife. The kibbles are typically cooked, for example in an oven. The cooking time and temperature may be controlled by the electronic manufacturing instructions. The cooking time may be altered in order to produce the desired moisture content for the food. The cooked kibbles may then be transferred to a cooler, for example a chamber containing one or more fans.

The pet food manufacturing apparatus may comprise a packaging apparatus. The packaging apparatus typically packages the pet food into a container such as a plastic or paper bag or box. The apparatus may also comprise means for labelling the pet food, typically after the food has been packaged. The label may indicate the type of dog that the foodstuff is suitable for (i.e. Labrador Retriever), and/or the ingredients of the food.

*Use of the foodstuff*

The foodstuff of the invention is for use in preventing liver copper accumulation in a dog. Therefore, it is for use in preventing a disease or condition associated with high liver copper such as copper-associated chronic hepatitis, cirrhosis or liver failure. Accordingly, the foodstuff of the invention can be used in a method of preventing liver copper accumulation and a disease attributable to liver copper accumulation in a dog having genetic inheritance of the Labrador Retriever breed, comprising feeding the dog the foodstuff as described herein, wherein the dog has no clinical symptoms associated with liver copper accumulation. The foodstuff can also be used in a method of preventing copper-associated chronic hepatitis in a dog having genetic inheritance of the Labrador Retriever breed comprising providing the foodstuff of the invention to the dog. The foodstuff of the invention is typically for prophylactic use in preventing the accumulation or copper in Labrador Retrievers. It is also typically for preventing copper associated chronic hepatitis in Labrador Retrievers.

Preferably, the foodstuff of the invention is for use in preventing the accumulation of liver copper in a dog determined to be susceptible to liver copper accumulation by the genetic test described herein, wherein the dog has no clinical symptoms associated with liver copper accumulation.

The use of the foodstuff of the invention may comprise providing a source of zinc to the dog. As described herein, zinc may be provided in any suitable form to the dog. The foodstuff containing the low level of copper of the invention may further comprise zinc, for example at a concentration of at least 120 mg/kg dry matter. Alternatively, zinc may be provided in the form of one or more separate foodstuffs or supplements. The use of the foodstuff of the invention may comprise providing a zinc supplement to the dog. A zinc supplement could be provided at any time, i.e. separately, simultaneously or sequentially to the foodstuff of the invention. The zinc supplement could for example be mixed with the foodstuff of the invention before it is provided to the dog, or it could be put into the dog's drinking water.

The foodstuff of the invention may be provided to the dog one or more times per day. The foodstuff is preferably provided in place of the dog's conventional food. The method of preventing copper accumulation or preventing chronic hepatitis may be used for an indefinite period of time, i.e. throughout the dog's life.

The invention is illustrated by the following Examples:

## Example 1

*Elucidation of SNPs associated with susceptibility to copper accumulation*

120 Labrador DNA samples were genotyped across more than 22000 SNPs. There were 72 dog samples from high copper dogs (liver levels of copper above 600 mg/kg) and 48 dog samples from normal copper liver levels (below 400 mg/kg). The data was analysed using pairwise comparison between every possible pair of dogs. Data was ordered according to support of a disease informative locus. Data from the best three genomic locations was used using Boolean operators to find the best fitting markers linked to high copper levels. Results of a simple Boolean model using the three locations are given below:

**Table I Results of simple Boolean model using the genomic locations CFA8, CFA32 and CFAX**

| CFA8 (GOLGA5 gene region) | CFA32 (UBL5 gene region) | CFAX (ATP7a gene region) | % of dogs with this pattern of alleles that have high copper | |
|---|---|---|---|---|
| 1 | x | x | 69.0% | |
| 1 | 1 | x | 72.3% | |
| 1 | 1 | 1 | 81.5% | Of the 27 dogs with all three alleles, 22 (81.5%) have high copper |
| 1 | 1 | 0 | 60.0% | |
| 1 | 0 | x | 64.9% | |
| 1 | 0 | 1 | 77.8% | |
| 1 | 0 | 0 | 60.7% | |
| 0 | x | x | 36.1% | |
| 0 | 1 | x | 55.6% | |
| 0 | 1 | 1 | 42.9% | |
| 0 | 1 | 0 | 63.6% | |
| 0 | 0 | x | 16.7% | |
| 0 | 0 | 1 | 50.0% | |
| 0 | 0 | 0 | 7.1% | Of the 14 dogs with none of the three alleles, 1 has high copper |

The key to the binary values in Table I is as follows:

Genomic location CFA8 (GOLGA5 gene)

1 = if there is an AA genotype at SNP BICF2P506595

0 = if there is any other genotype at SNP BICF2P506595

Genomic location CFA32 (UBL5 gene region)

1 = if there is a GG at BICF2P772765, a CC at BICF2S2333187 and a GG at BICF2P1324008

0 = if any of those SNPs show a different genotype

Genomic location CFAX (ATP7a gene region)

1 = if there is an AA or an AG at BICF2P591872
0 = if there is a GG at BICF2P591872

In all locations, X = unused alleles.

Table I represents the binary conditions of alleles at three genomic locations. At genomic location CFA8, one SNP was used (SNP 1). At genomic location CFA32 three SNPs were used (SNPs 2, 3 and 4). At genomic location CFAX one SNP was used (SNP 5). The binary values are indicative of a dog having alleles that are indicative of susceptibility to copper accumulation ("bad" alleles). For instance 000 represents not having any of the three bad alleles. 111 represents having all three bad alleles. The Xs are unused alleles at that gene. The lines 1xx and 0xx show the power that a one gene test only using the SNP in the GOLGA5 gene would have.

Table II shows that dogs with more of the indicative alleles have higher copper concentration on average. We can also see the number dogs with each pattern:

**Table II**

| Gene Combination | Average amount of $Cu_2$ (mg/kg) | % of dogs with this pattern of alleles that have high copper | Number of dogs with pattern |
|---|---|---|---|
| 111 | 1253.09 | 81.5% | 27 |
| 110 | 733.40 | 60.0% | 20 |
| 101 | 1138.90 | 77.8% | 9 |
| 100 | 737.84 | 60.7% | 28 |
| 011 | 502.27 | 42.9% | 7 |
| 010 | 670.83 | 63.6% | 11 |
| 001 | 450.00 | 50.0% | 4 |
| 000 | 332.47 | 7.1% | 14 |

**Table III Position and sequence of SNPs used for results in Table I and II**

| SNP | SEQID NO: | Chromosome in canfam 2 | Location in canfam 2 | Gene containing or close to mutation | SNP Sequence<br>SNP = [wildtype base/alternative base] |
|---|---|---|---|---|---|
| BICF2P506595 (SNP 1) | 1 | 8 | 4886813 | GOLGA5 | CTCAGAACTAGATAGGCTAATAAGTGATAGGCCTTGTGTTTTC CTAGAGTGTGCTTTAAA[A/G]GTTTCTTAAGCTAAAAAATTA CATTCGTGAGAAAATTGAAATAAAAGGAAAACAGTCATG |
| BICF2P772765 (SNP 2) | 2 | 32 | 39278300 | UBL5 | TCTCAGATACTTGATAGCCAGCATTTCCCCCCATTTTCTTCCA AGAGCACGAAAGCATAG[A/G]AATGATATTACATCTCGTATG GTGAATGTGACACAGCCGTCAGTTGCGTTAGCTCTGCTT |
| BICF2S2333187 (SNP 3) | 3 | 32 | 39390236 | UBL5 | TATTACCCTGCTCTCCAGCCACTCCTTTACCTTCCATTAGCCC ACACCTGCTCTACACAC[T/C]ATTGCTCATGGAAGCCTTGCC ACGTCCAGTCGCCACTCTGAAATGCCAGCATCCCTCCCA |
| BICF2P1324008 (SNP 4) | 4 | 32 | 40043909 | UBL5 | GACCTGACAGATTATGTAGACTTTGTTTTCAAAGGGAGCACCT GCTGGATATACAACATG[A/G]CACTAAATTGTGCTCCACATC CTTGGCAGAGGTGGGGGGCGGGGCACAAAGGAAGAAACC |
| BICF2P591872 (SNP 5) | 5 | X | 62989720 | ATP7a | GGGCCCAGCAAGTGGCAGAACTGGGAAGACCCCCTCTTCTTCC GCCTGGAGCAGTGGTGT[A/G]GCAGCACACCACAGGAGTCTG AAAGGGTGGGGAGTCCAAACGGGAACATATACCTGAGAT |

Table IV Position and sequence of further SNPs indicative of susceptibility to liver copper accumulation

| SNP | SEQ ID NO: | Chromosome in canfam 2 | Location in canfam 2 | Allele Frequency | Minor Allele Frequency | SNP Sequence SNP = [wildtype base/alternative base] |
|---|---|---|---|---|---|---|
| BICF2P1246154 | 5 | X | 47335181 | 0.999507 | 0.000493 | GGCAACAGGGACAGGCTGCTGGGCCACACACTCACCCACACT AGGAGACAAGATCCTCCA[T/C]ATCCTGGGTCTCTATCAGT CAATCACCTAGACCAGTGGGCCAGAGGACAGGGTCCAGCTG |
| BICF2P463335 | 6 | X | 44401786 | 0.000493 | 0.000493 | GTTGAGAGAGATCATACAGATTCATGTGGCAGGTGCACACTT TTTCTACCTCTTACAACG[T/C]ATTCTCTCTGGCCATTCCT TCTCCTGGGTCCCAAAGTCGGAGAGCTTAGCGGGAGCCTAG |
| BICF2P1246989 | 7 | 8 | 4149835 | 0.999506 | 0.000494 | ataagttcacattttgGTGTTTCAAGTGGACATGAATGGAGG GGAGGGCCCTGTTCAATC[T/C]ACTAAAGTGTTTTTTCATC TTGTTTTTGTGGAAATCAAATCAAGAAGCAGAGTTTATGT |
| BICF2P723557 | 8 | 8 | 3406227 | 0.999014 | 0.000986 | ACTCTCCCGATGTGGGCACCATATGGTGGACCACTTTCTGTG TGAGATGCCTGCTCTTAT[T/C]GCCATGTCCTGTGAAGACA CCATGCTGGTGGAAGCATTTGCCTTTGCCCTGGGTGTTGCC |
| BICF2S23427298 | 9 | 8 | 5393517 | 0.999014 | 0.000986 | AATCTAAGTAGACTGAGTGGTCACCTTCAGCGCTCAGACCTG AGCATACAAAGCATGGAA[A/G]GTTACTGTGATTCAGCTGA TGTAATGGAATGAAATAAATATAAGAGTTTGGTAACCTAAT |
| BICF2P312189 | 10 | 8 | 5773958 | 0.999014 | 0.000986 | TGGAGAGTGCTGGCAGGCAGGGGCAGGCAAACAACAATAGCA AAGATCTCTTCCACGCTT[T/C]TACTTCCTCAAAAGTCCAA GCCCTCTTAAGATCGCATTTTCTTAGTGACCTTCACTCTAA |
| BICF2S24321583 | 11 | X | 56410647 | 0.999014 | 0.000986 | TTCTTTGCTAGGCCAAGGGCAGAGAATGCATGCCCCCCCTTA CCTCCCAGGGCCCAAGAG[C/G]CATCCTGAGCTGAGTCTAT GGCTCCTGGTGGGGGGCGGCTGTGGGTTGGGGGGGCACAGA |
| BICF2P1273450 | 12 | 8 | 3160594 | 0.999013 | 0.000987 | ggtgtcaccaatgccagcgagcaccagctggagggaacagga cacaggtcctccgtcCTG[T/C]GACACTCGGATCTGGGGCT TTGCCTCCAAAACGGAGACCATGCCTGTCCATGGTTCTACG |

| SNP | SEQ ID NO: | Chromosome in canfam 2 | Location in canfam 2 | Allele Frequency | Minor Allele Frequency | SNP Sequence SNP = [wildtype base/alternative base] |
|---|---|---|---|---|---|---|
| BICF2P1439540 | 13 | 8 | 3771142 | 0.998521 | 0.001479 | CTCTAGAACCCTTCAGGTAGACTACATTCACTTTCTACTACA ACTTCATCACCACAACCA[A/T]CTCCCAGTAACCCCCtttt tttcttctccttttttattttttccttctttttgctcgtc |
| BICF2P506204 | 14 | 8 | 4191144 | 0.998521 | 0.001479 | TCCCATGGGTTGAAGGATATCTGGCAGACGGCTCCAACTCCA GTAAAGCCTCAGGCCTCA[A/G]CCAGGAGTTCCCCGGGGCT TCATTCCCATCCCAGACTTTGCCCAGGGCTGATTTGAAAGT |
| BICF2P380732 | 15 | 8 | 3299879 | 0.998519 | 0.001481 | TCTTCCTTGCAGATTGGATGGCTGTAGCCTCACCTCACACTG TTGCTGGGATCTGTCCAC[A/G]CTTCTGACCTCCAGCAAGA GCCTCCGGGAGCTAAGCCTGGGCAGCAATGACCTGGGAGAT |
| BICF2G6301602 0 | 16 | X | 73980557 | 0.004955 | 0.004955 | TATTGCTAGTAAAGCCAAACTTTCTATTCCACAATTATAAAC TCATGGAGATGGTAATTA[T/C]AGTGCATTATTTGTCAAAT TTTATTATTTTTTCAAATCCCAAAGAAAATGTGATATTCTA |
| BICF2S2362356 9 | 17 | 32 | 38362784 | 0.994576 | 0.005424 | AAGAACAAGGATACAATCTAAGTGATAATCATCCAGCATGTA CTTGTCCTGTTTTCAGAT[T/G]ATCAGCTTAAGTCAAGAGG AATTTTTAGTGCTTACAAATATTTCAAGTGATTTTTCCAGA |
| BICF2P216837 | 18 | 8 | 7474389 | 0.012327 | 0.012327 | TGAAGGGGTGCTACTCAGGGCTCTTCATTTAACCTTCCAGGA TGTTTTCCTATGTACTCA[T/C]TCTTCCTTTTGGTTGCTCC TTCTTCTTGCATTTCTTTATCTCTTTACAGAATCATCCAGG |
| BICF2S2292214 6 | 19 | X | 75388683 | 0.986193 | 0.013807 | acaaccctaaaatttcagtgattcagtacaacaaaggtttat tATAACCATTCAGGGATC[C/G]AAGTTGGTAGAAACTTCAC TACAATACCTGCTTCCAGTCAACAAGACAGAAAAAGAAAAA |
| BICF2G6301571 4 | 20 | X | 74415223 | 0.01382 | 0.01382 | GCAGGGTTGATATATAACTAGTATGCATTAGGTAGACACCTA TTTTGATTACTCACTATT[T/G]TAATATCAGCCTGGTAGTA AGAACCAAATCTATTATGTAAAGTGCATAGAGAATTGaaag |

EP 2 195 460 B2

19

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2G63015674 | 21 | X | 74439123 | 0.0143 | 0.0143 | CTAGCTAGCCACCCAACTCCCCACATGCCCAGAGTCATCGTT TATCTTTTCACATCAGCA[T/C]TACATTTTGGCTTGCATTC AAACATTAGCCCATTTTTTTTCCTTTTGTTTTATTTATAGA |
| BICF2P426463 | 22 | 8 | 5833993 | 0.015286 | 0.015286 | TTTTCTCTTTTTCCATAAATGCTCTGGGCTTATTTTCATTAT CTAGTATTTCTCTTCTGA[A/G]GCTAACTCCCAAAGAGTTT TGTGCATCCTTATTTCCATCACAAGGTCAATGTACGAGTTA |
| BICF2S22926688 | 23 | 8 | 7502279 | 0.015779 | 0.015779 | GGGCCCAAGGGCTGAGGATCTCTGTACCTTCTGCTTCTTGGC AGCCCAGGCTGGGTAGCA[T/G]TTCTTGGAAGAGGATTTCC CATGAGTTGTTAACAGAAGGGCGGGCTTCCAGGCGCTGCTT |
| BICF2P1113947 | 24 | 32 | 38074100 | 0.981169 | 0.018831 | CATCTTTGCTTGGGGCCTGGGGTTTTTATTGAGGATTGTGAT CTGGTGTATGTGTCTCCT[T/C]AGGCATCCAGAAACCATTC AGAACAAGAACAAGCGTCCAGGTATCCTCTGTAAGTCACTT |
| BICF2P342874 | 25 | X | 44861101 | 0.020217 | 0.020217 | ACAAACCCTCAGACCCAGATACACAGTATCATGTGGACACAG ACATGTAACACCAAAATG[A/C]CCAACATCATGTGACTACA GGCCCTAAGCAACTAGGTGTAACATCACTTGGGTATGGGCC |
| BICF2P1171925 | 26 | 32 | 36457625 | 0.022189 | 0.022189 | AATGCAGTAATACATGTAGCTAAACCTAACCATCAGAGTCTG TTCTATCCTTCTACAAAA[A/G]TAGGGTTGGAGCTGAGCAC ATAGGTAGCATACATCTAGCAAAGTTTTTGCCTTCAgatt |
| BICF2G63017200 | 27 | X | 71984532 | 0.025641 | 0.025641 | ttgtggggtcaggtgagttatggacccctccctactcttctg ctatcttgccccCTACAG[T/G]GGTTGCTATTTTGATGTAA TCACAAAACGACCTGGCAATAAAACCTTTTTCTAATTAggg |
| BICF2P1286548 | 28 | X | 57448138 | 0.026423 | 0.026423 | GATGCAAGCTGGGACAGAATAAGGTACTGGGCTGTGTCAAGC CCCAGTAAGAGAGGAGCA[T/C]TGTAGGGTAGTTAGGATGG ACTTAATGGAGATGAGTCCTAGGGAGCCACACTCAGAGTTA |

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2P790089 | 29 | 32 | 38885957 | 0.0286 | 0.0286 | TAAACACCCCCAATCACTACCATCCTCACACCTAAGGATACA CAATGTGTCTACTTTATG[A/G]TATGTCTTTACTATTCGTT GCTTATGAAATTTTATTCATTAWCTAAAACAGGGAAAAAAG |
| BICF2G63016713 | 30 | X | 72619011 | 0.9714 | 0.0286 | TATAGYTGGSCAATTAAATCTCCTATTCTTTTGTCTCAAAGG ATATTTGAAATTACATAG[T/C]TCTTTTCTCATATAAAACC TACCATACAATCATTAGATGATCCTTCTTAGTTAATTTTTT |
| BICF2P276536 | 31 | 8 | 3149437 | 0.966436 | 0.033564 | GATGCTGTGGGCCAGTCCAGAACCCACCTGAGAGAAACAAAC AGGCCTCTTTGCCAGCAG[A/G]GCAGCGTCAGTGTCACCCC TGTGACATGTCAGAACCTCCCTGAAAGTTCATCTAACCTCT |
| BICF2G63015658 | 32 | X | 74531965 | 0.963018 | 0.036982 | GGCTCAGAAGAAAAATCAGCCCAGTTCACATCCAATGTTTCC ACACATCTAATCGTCTTG[A/G]GTTCAGAGGTAGATGTGGT ATCACTTAYATGGACACATATAACAGCTGGCCCCCACCTCT |
| BICF2P308749 | 33 | 8 | 7325380 | 0.962032 | 0.037968 | gtttcagttaattatagtccttactggatccgattgctgtgg cgctaaaatgaAAGAAGG[T/C]Agggtacctgggtggctca ggggttgagaatctgcttttgactcaggtcatgatcccagg |
| BICF2P872820 | 34 | 8 | 6388554 | 0.956114 | 0.043886 | CAGAGTAGCATTATTTTCTGCTGTATGAGGACACTTTTGTTA TATCCACAGTGGACAGAA[A/G]ACTGGGTTTTAGAACATGC TCAATTGAAACAAGACTGAGGGCTCACAAATTCCTGCTCCA |
| BICF2G63016210 | 35 | X | 73592920 | 0.955084 | 0.044916 | TTACTTATTCATCTGAGACCAAGGCCACTGTGGTGAACCTAC AAAGCCTTACAAAGCAGG[A/G]CCAGAAGGGCACATAAATC ACTTGACTAACATTTGGTCAAAATAGCTCTTGGGCTCTTTT |
| BICF2G63016209 | 36 | X | 73593955 | 0.049456 | 0.049456 | ATAAAAATAAAAGAGCTATTAATAAGAACTCATAAAATCTAC ATAAATATAGTAACAGGT[T/C]AATATTCCCAGCATATTTT TACAAATCATCTATAAAGAGCATGAGAGCATATAGGGATTA |

| SNP | SEQ ID NO: | Chromosome in canfam 2 | Location in canfam 2 | Allele Frequency | Minor Allele Frequency | SNP Sequence SNP = [wildtype base/alternative base] |
|---|---|---|---|---|---|---|
| BICF2P1149405 | 37 | 32 | 41212550 | 0.941321 | 0.058679 | GCAACAACCTGGTTTGTGTGTGGGAAGCTAATGCCTCCCCAA ATGCAGCAAACTCTCCTC[T/C]TGATTTTAGAAAAGCAGTT TAGTTACAGGCAAATGCATACATGCATGATAAATACTACTC |
| BICF2G6301617 3 | 38 | X | 73672050 | 0.940828 | 0.059172 | GATTTTATAAAACATGATGACCTTGGCATTTATATAGTAGAT ATTACTACTCTGAAATTC[C/G]AGGAAGTATGATCATAAAC TCACACTTAATCTGGTAGAAGTATGGACAATGTATCAAAGG |
| BICF2P401962 | 39 | 8 | 4495597 | 0.935897 | 0.064103 | CTTGGTTGAGTTAAAACATTTGCCCATGCAATTTAATGCATG TCCCTGTGGGGTTGGAAC[T/C]GACGTACACCCGAGCCAAC AGCCTTTCATGGCAGACGCCATCAGGCAGGTGACCCCACC |
| BICF2P991264 | 40 | 8 | 3165755 | 0.071992 | 0.071992 | CCTTCCACACGCTCAGGTTGGCACGGAGGGGGTGTCCTTGCC TGAGGGGTCCTGGCACAG[T/C]CATCAGGGCACACAGCTGA TAACCCAAGGGAGCAGTAGGCAAGACCTCATGGGCGCCGGG |
| BICF2S2323084 7 | 41 | X | 58531292 | 0.079389 | 0.079389 | ATTCTCTTTGCTGTCTCCTGTATACAGAGATAAAAGCAAGAG TTTTCCCCTTCAGGTTTC[T/C]GAAACCCAGCTTCCTTTAG ATTTTAAGGGGTATTCTGTGTACCCATTTCCCACCTTCTGC |
| BICF2P1252842 | 42 | 8 | 4618608 | 0.919132 | 0.080868 | GCGGGTTGGGACCCCCCCTTCTGCTGCTCCCACTTCAGAGTT GTGGCGTCACTAAGATGA[C/G]ACCTCATGTCGGGAACCTG AGAGTCCCTCGGGAGTTGTGcagggactgtagccgacctat |
| BICF206301719 8 | 43 | X | 71984983 | 0.913947 | 0.086053 | ACATATGCACAGTGAATCGTGGATTGTTGTGTTTGATTTCTT ACATGATACAATAAAAGG[A/G]AAGTAGTTGAAGCAAAACT TTAGTTTAAAGGAAACAATTTCTCTATCATAATGTTCAGTG |
| BICF2P1364202 | 44 | 8 | 3175135 | 0.910256 | 0.089744 | CCCACAGACCCCAGGTGCTGACCACAGCAGCCACTTGGGCCC CCAATGCAGGAGACACCT[T/C]GGGAATGAAGGGGACAAGG CCAGCTCAGGCACATCGTCAGTGCACCTGATGGGAAGGCCG |

EP 2 195 460 B2

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2P963708 | 45 | 8 | 5472668 | 0.095945 | 0.095945 | ATCTGATCCTAGCCAATGGAAAGCAATTTGAGATAGGAATCA TATCTTGTTTTGGTTTAT[A/G]TGCTTTCTTTGGAGTTTTG CACATCATAGATAACTGTAAATTTGTAGAATAAATGTTTGA |
| BICF2S2293948 1 | 46 | 8 | 7696228 | 0.098619 | 0.098619 | GTCAATGCCATTAACCTGGCGAAGCTGCTCGAGCATCCACTG CGATCTCCGCACGAACGA[T/C]GTGGAGCCTTCAAACTGTT TGACCTTCGTGATGGATGCTTGTGTGGGTTTCTTGTTTGTC |
| BICF2P1028186 | 47 | 32 | 40758922 | 0.107495 | 0.107495 | ACTGGTTAATAAGACTTCACAGATTTTATCCATCATGTTGAT TATCTGTATATGTATTTT[T/C]TACCACTTAGGATAAAGTT CTGTTATCTGTAATTGATTCCAACCAGCATGTTTGCTCCAA |
| BICF2P19238 | 48 | 32 | 40849057 | 0.892012 | 0.107988 | CTTCTTCTTTCCCATTGGATTCTTTCATCAATCGTAGGTAGT TCTTAATGAAGATCTGTG[A/G]TAAAGCCATTCATCTATTC ATTCAACAAATGGCATCACAGAAAAGAAAAATAACCTTTAT |
| BICF2P247312 | 49 | 8 | 7825200 | 0.112426 | 0.112426 | GGGACACATTTCTGGACAGACCTCTGATCACACTCACAGGAC AGCAAGAGGAAGCTCTGG[A/G]TACAAGTACAGGGAAAAAA GAAAGAAATGGTCACAGGGAAGCTGCCGCAGGAAAAAGGTA |
| BICF2S2301711 8 | 50 | 8 | 7615543 | 0.881164 | 0.118836 | GGGCAGATCCTCAGTGAGTATTGGCTCATGTTCTCCGAGGGA AGTAGAGTCCCAGAAGAA[A/G]GATGCTAAGGTGCCAAGAT TCCTGAGCCTGTGTGTGGTACAGTCACAGCAGTACTCCTGA |
| BICF2P132419 | 51 | 32 | 35699747 | 0.874506 | 0.125494 | TCATCTCCATTTGTAATAGAAACCACATATATAGAGAGATTG GATTATTAACCACTAAAA[T/C]GTAGCCACTCAAGGGGAGG GGGGGAATGCATTTGGTTTATTTCCCATGTCAAAACAGAAT |
| BICF2S2311591 1 | 52 | 32 | 40712955 | 0.873393 | 0.126607 | AACACTGCTAATAAATATTTATAATGGTTTGAGGAAAATATC AGGTGTGAGATGTCTTCA[T/C]ATCATATAATATATCATAA TATCCTCTAAAAAAGCTCTAAGCATAGGTCTATGGAACTCA |

EP 2 195 460 B2

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2G630531773 | 53 | X | 43502595 | 0.127219 | 0.127219 | AAGCAATCCAGGAGTCTTTCTCCGGGTAGCAGGCTCGCTTTACAGGTTAAGGCTGGATGA[A/G]AAGGAAGAACCTGAGCTTCAAATTATCATCTGAGTAGAGCTGATACCCATGGTTACATTA |
| BICF2G630587826 | 54 | 32 | 38771348 | 0.127838 | 0.127838 | GATTTTATTCTTTACTTTGATTTTTTTTAAGTTTTACTATGATATTCAATATGATTGTGG[T/C]TCATGAGATTCCTCTTTTTAGCTGTATCATTAACTACAGAGCGTTCTCAAATATTTTTCT |
| BICF2P1007047 | 55 | 8 | 4812890 | 0.87092 | 0.12908 | GTGGCCGGAGGGGGTGGGCCCTACTGTGGCCCAGCTTCACGTCCCACTGGCCAAACATCA[A/G]GATGCAGACACCCAGGTCCCTTGTGCTGCCTGCTGAGGCTAGGAGCAGCGACTGGAAATG |
| BICF2G630531804 | 56 | X | 43317321 | 0.869329 | 0.130671 | GATGGGAGACCTCATACACATGCAAAGATCACTATTAAAGACTCTCGAGCAAAGATCGAA[T/C]GGACTGTGGCAAGCTGCCGCGCATGCCAATCAACAAATGCCTCCGACCATGGATCTAACC |
| BICF2S2363287 | 57 | 8 | 5656863 | 0.866371 | 0.133629 | CAACAAGGTTTTTAAGGTTCTTTTCACTACCTTCTTCTTTTTGTACTTGCTTAGGACACC[T/C]GTATGTCTTCACAATATCACCTGAAAGTCCTTTAGGAGATATACTCAAAAAATAAATAAA |
| BICF2G630155587 | 58 | X | 75321307 | 0.865385 | 0.134615 | caacctgagctgaaggcagacactcaactgttgagctacccaggtgtaccAAACACATCT[A/G]CTCTTAACCAAGCTTATTCTTTGCTATATTTGGCAAATTGTGGCATGTCTACAGTACTCA |
| BICF2P482693 | 59 | X | 43587959 | 0.864892 | 0.135108 | ATTCCCCATGTTTGAGGAAATCACAGGAGCCACTAGGAAATCAACCATTTCCCAACCAAC[T/C]TGATGATTTCCTGATCCAAAGGTTCTCCCAGGACAAATATGAGGTAGCCTTTCACACTCT |
| BICF2P940430 | 60 | 32 | 40921126 | 0.136364 | 0.136364 | CAGTCTTGTAGGAGAGTAGATTGACTCACAGAACTGGCAAGATTGGGAATCTGAGCATTG[T/C]CACTTGAGTCTTAAAACGTTTACGATTTTATTTCTAGTATTTCAATAAGAAACACATTCT |
| BICF2P786384 | 61 | 32 | 36389913 | 0.136723 | 0.136723 | GAATACATTGCCAGAATAATTTCAAGTTCTCAAATCTCAACTAATAAGATTTTCGTTAAA[T/G]AAGGCATTCAATCATCACTTACTGACAACCCACAAAATTAGGCACTGATGAAAAATTAGC |

EP 2 195 460 B2

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2P1340243 | 62 | 32 | 41050914 | 0.150394 | 0.150394 | AAGTTAAGATATTCAAGAAAGAGAAGAGAGTGACTGAGCTAA AAAGAAAATCAGATCTCT[T/C]CCAGGCTTTAAAATAATCT CCACAATACTGGGCAATCCATGTAGTCTCCCCAGTTCCATT |
| BICF2S2362644 5 | 63 | 32 | 36617978 | 0.153846 | 0.153846 | CTCAAAAGGAAAAGCCTGTGGAAAGGCAAAGAGGTATGTGAA AGAGGTAAGTTCAAAATG[C/G]TGACATGACCAGTGTACAT AGATTACAGGGTACTTGGAGGAGCAGTGAGAAAGGAGTCCA |
| BICF2P161586 | 64 | 32 | 37795702 | 0.156312 | 0.156312 | TTCTATGAAATAGCTACCATTCTGGTTGGTATCTTCTGTTGA TTTAGATGATGAAGGAAG[T/C]ATAAGAAGTAAGGCTTATG AGTTTATAAAGCTTTAGTTAAAGCTTTGATTGTGACAAAGC |
| BICF2P579617 | 65 | 32 | 36631235 | 0.162389 | 0.162389 | AGAGGAGAAACACAGCTAAAAACTTTTTTACAGACTGGACA AAGGTGCTTACACTTTTC[A/G]TATTgggcagaatgagggg atgaaaacaccagtggtcttttttgaagccacacaaattcag |
| BICF2G6301628 0 | 66 | X | 73386098 | 0.835968 | 0.164032 | AGGATGAATATTTATTAACAGTAAATATACATTTTTATTGTT CTATATACTCTAAAGACA[A/G]TTGTAGACAGTAAGATATA TCAATTTTAGAAACAGAAATAATGTTAATTGTATAATATGG |
| BICF2P721687 | 67 | 32 | 40771787 | 0.829389 | 0.170611 | CAGGGATTCCTAAAGGGTGACATGGTATGGTCTAACACTTCC TCACTGTCCTTTTCCCAG[A/C]TGATATAAGAGGAGGACCA GAGAGACACATAAACTGTCTGAGTCTTTAGCATTGTGATAA |
| BICF2P504739 | 68 | 32 | 37328946 | 0.827909 | 0.172091 | ACACTAATGGGTAGAGAATACACGTCCATCAGTCATCAATGT AATCTACTAACAGCCTCA[C/G]AGTCTGGCAGTTTTCAGTG AAAAGAGGAGTCATCTCCATTTATTCGAtcaatcagttgac |
| BICF2S2333187 4 | 69 | 32 | 39390236 | 0.825444 | 0.174556 | TATTACCCTGCTCTCCAGCCACTCCTTTACCTTCCATTAGCC CACACCTGCTCTACACAC[T/C]ATTGCTCATGGAAGCCTTG CCACGTCCAGTCGCCACTCTGAAATGCCAGCATCCCTCCCA |

EP 2 195 460 B2

| SNP | SEQ ID NO: | Chromosome in canfam 2 | Location in canfam 2 | Allele Frequency | Minor Allele Frequency | SNP Sequence SNP = [wildtype base/alternative base] |
|---|---|---|---|---|---|---|
| BICF2P772765 | 70 | 32 | 39278300 | 0.816075 | 0.183925 | TCTCAGATACTTGATAGCCAGCATTTCCCCCCATTTTCTTCC AAGAGCACGAAAGCATAG[A/G]AATGATATTACATCTCGTA TGGTGAATGTGACACAGCCGTCAGTTGCGTTAGCTCTGCTT |
| BICF2S2318354 | 71 | 32 | 35849858 | 0.18787 | 0.18787 | ACAGGAAGGAGAACTGAGCATCAAGAGAGTTCAGAACATGAT CATTGGGTCAGTTTGTGG[C/G]TGCATTAACTTTTCCCCAA AACAGAAAGCAACAGAGACTTCTGTAGGTCAGTCAACAGTG |
| BICF2G630588054 | 72 | 32 | 38521693 | 0.810052 | 0.189948 | TTACCATTACTATAACCCAAGTTATAGTATACTATAACCAAG TCCTTAATTGACTTGATG[T/C]TTGTGCAGCTGATTTTAAA TCTATTTAGAATAATAGTTTACTTGTGACAATTCATATTAA |
| BICF2S23313445 | 73 | 8 | 6343006 | 0.809665 | 0.190335 | TTGGTCGACTGACTGATTGGTTTTACTGTGGAGGAAAGAAAA GGGAATTTTCCCAAAGAG[A/G]ACAGAGAGAAAACATGGAA TTGAGCAAAGGGAGAATAGAGAGACAGGGCAGCCACTGAAG |
| BICF2P675334 | 74 | 8 | 4477476 | 0.19428 | 0.19428 | TGCCTTATCCTCCAGCTCCTCCCTCACCATCTTGGAAACTAG CTCAAATGTCACTGGTAC[T/G]TGTCTTTCTTTTGATCTTT CTGAAAGACAAACATGATCCCATCACCTCTGCCTTTAGAAC |
| BICF2G63017409 | 75 | X | 71722644 | 0.804241 | 0.195759 | ACTCCTAAGTAAAAGTTAAATTAACAGATTTGCCATCAAGTA CCTTGCCCATTTTTCCTA[T/C]AGATCGACTTTTTACTGGA TGATCCCCTTGATAATAATCTTGATCTATGTTTTAATTCCA |
| BICF2P798346 | 76 | 8 | 4651519 | 0.195759 | 0.195759 | ctggtgggcttgtcaggggcaggatgttgtgtggtgagcaca gaattaaaactaggaGCT[T/C]gaagcgcctgggggggctca gttggttgacggactgccttcatctcaggtcatgatccctg |
| BICF2P1150684 | 77 | 8 | 7652070 | 0.802761 | 0.197239 | CATACAGCGAAGAGATAAAAACACAGGATGCTGGGCTCACGA CCATGACCGGAAAAGGAC[A/G]GCGAGGAAAAGCAAGTATG AGCAGCCCAAAGTCCTTTTTCCAGCACTGGCCATAGGAGGA |

EP 2 195 460 B2

26

(continued)

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2P1348758 | 78 | 32 | 36083895 | 0.801579 | 0.198421 | CAGAGATGAGGAATCAGACTCCTCGTCCTCTGCTTCTCTACA ATGGCTCATGTTCTCCTT[T/C]CCCCTCAGCTGTTGCATTA ACAGAGGTCAACCCATTCTTCTAAATTTAAATCTCCCAGAA |
| BICF2G63017599 | 79 | X | 71555277 | 0.198617 | 0.198617 | AATCAAACAAGTGCTAGAACATAGAACAAGTGGCTCATCTTT TCCCCAAATGTCTGGATA[A/G]GAAAAAAAAATCTAAACA AATGCTAGATGTTAAGTATCTGAAATGATCAGCCCATGAAA |
| BICF2G63016090 | 80 | X | 73800072 | 0.200197 | 0.200197 | TCCATACCAGTCCTTGTTGTCTACCCCGAACTTCACCTCTCT AGGCACAGACAGCTCTAA[A/C]TTTCACTCATAGGTATCTT ATGCTGACCTGGCCTGCCTCCtgttttgttttgttttgttt |
| BICF2S23524027 | 81 | X | 64785623 | 0.79931 | 0.20069 | CAAAAAATTCCCTGAGCCCAGCATCAAGGTACCTGGTTTGGA GTGGGTGGGTCCTCAGAA[A/C]GAATGGGTGTGGTGTACAT TTAGCAAGTTATGTAGCATGTGTCTGTGTAGTCTCACCTCT |
| BICF2P591872 | 82 | X | 62989720 | 0.795252 | 0.204748 | GGGCCCAGCAAGTGGCAGAACTGGGAAGACCCCCTCTTCTTC CGCCTGGAGCAGTGGTGT[A/G]GCAGCACACCACAGGAGTC TGAAAGGGTGGGGAGTCCAAACGGGAACATATACCTGAGAT |
| BICF2G630587712 | 83 | 32 | 38968302 | 0.794379 | 0.205621 | atataatataacttatttaaaatatttGAAGATATTTCTATA GTTATGCTCTACCATTTG[T/C]TATTATAAGATTTCCAACA GCTTACTTCTTGTATGAAATTAATTTACCAGCCCCTCACCT |
| BICF2G630587722 | 84 | 32 | 38964413 | 0.792899 | 0.207101 | CCCTATTCTATAAACATTCCCTCTCTGGCCATCCTGTCAAGT GGGCCCTGACAGTGTGCC[C/G]CAGAAGCTCCCTAGCCTTT GCCCATTCCAGCTATGGCTAGCCTGCCACCAGCCATACACA |
| BICF2G630185557 | 85 | X | 66396513 | 0.218164 | 0.218164 | CACTGTGAGGTCTGAATGGAGACATTCATGATAGACTCCAGG ATTTTCCCAGCTATTAAG[T/C]CATGGGCCATAAACTGGAA CACTTGGAAACAGTCCATAGGTTCATATTAAAGAATATGTT |

EP 2 195 460 B2

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2P652606 | 86 | 32 | 37855796 | 0.776134 | 0.223866 | GCAAAAGGAACATGAGTTCTGATCTTCTGTAAAGGAGGCTAA TTTACTAATGGTCATAAC[T/C]GTGGcctgagggtcaagtt tctaattaaacgtgcatcttggggYggactagaatactttc |
| BICF2S2331279 9 | 87 | 32 | 36791310 | 0.224852 | 0.224852 | CAAGGSCCAGGTACCCTGAAGGAGTCCGCTTCACCCAGGCAT GATGTGTTTGACAGTCTT[T/C]GTAATTGATACAGCCATTG GCATCCTCTTGCGGCCAAYATCAGCTCCACTTCAACCTCGG |
| BICF2S2303948 | 88 | 8 | 5896281 | 0.773669 | 0.226331 | TGCAATGGGTTTTGAAATTAGAGGACATCACAGCAGAGTAGA ATGGTTTGGAACAGGGGA[A/G]TATGATTAGGATTAATGAG ATGAAAGAAAATTCTGGCTAGAGGGCTAGAAGAGCCATGGA |
| BICF2P506595 | 89 | 8 | 4886813 | 0.228304 | 0.228304 | CTCAGAACTAGATAGGCTAATAAGTGATAGGCCTTGTGTTTT CCTAGAGTGTGCTTTAAA[A/G]GTTTCTTAAGCTAAAAAAT TACATTCGTGAGAAAATTGAAATAAAAGGAAAACAGTCATG |
| BICF2S2313060 0 | 90 | 8 | 5180802 | 0.228304 | 0.228304 | GATACTTTGGGCTCTGGGTGGGAGCCAGCAGTGGTGGGGCAG GGCAGGAGTCCAGCAAGG[T/C]GTCTGGGCATACATGTCTG AGAGTAGGAAAACCACACCATTGCACCTTGCCTTTGACTTC |
| BICF2P1270451 | 91 | 8 | 5580117 | 0.229783 | 0.229783 | TCAAGGATCAGAAAAATAAAAGCAAAGAAAGAGGCAAAGAAA GAAGAAATGAAATACCTA[A/G]TGGCAGAAGTAGGCAGAGA AATAAAGGCTAAAAGAAAATGGCAGAGGATTGTTTGAAAGG |
| BICF2G6305882 67 | 92 | 32 | 37876000 | 0.23001 | 0.23001 | TATGTTATACTATTTTAGTATCTTAATAAATATGATTAGCCA AAATAGTTTTATCATCCT[C/G]AAAAGTGCAGCATATATTA TTTTCTATTAAATTCAGAATAGGTATAAACTAGAAAGCATT |
| BICF2S2312207 4 | 93 | 8 | 4965974 | 0.76999 | 0.23001 | ACAGCAGTTCTGAGGATGGACTCGCAGAGGCTCCTGACAAGC AGAATGACCAGGCCGAGC[A/G]GAAAGGTCAGTGCTGCCAG TCTAGCCAGAAGTGGGGGAGAGAGGATGTAGGAGCAGTACT |

EP 2 195 460 B2

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2P555643 | 94 | 32 | 40258722 | 0.230769 | 0.230769 | ACTGTACTCAAAAAAGTTCTGTTTGCCTAAATGGGATCAGCC TCTAATGGATGCCAGTGA[T/C]GGGAGGCTGTTCATCATCC CTTCGGGATAATTCAGAGCCTAGGCAGAGGCCCAGCGTTCA |
| BICF2S2325999 9 | 95 | 8 | 4990277 | 0.231732 | 0.231732 | TACAGGCCCCAGGAAGGAGCCACCAGATGCCCAGGACTGGGC CCAGGAATGATGGAGGCT[A/G]TACAGCTGGCTGCCTGCAC TGGCTGCCGCCCCTGTCATCCAGTGTCACAGAGCAGCACCT |
| BICF2G6301652 5 | 96 | X | 72989415 | 0.251482 | 0.251482 | AGACATTGCCAAGAAGTATCCACAATGAACAGTTTGAAGGGG ATCCAGAAAAGCACAGGG[T/C]CTACTTCCGCTGGATGAGC AGCAGTGAGAACCACAGTCAGGTAGGTCTTAAAGCAAAGTT |
| BICF2G6301955 2 | 97 | X | 60108249 | 0.737179 | 0.262821 | GCTTTGAAAACCAACAGGAAATACATCCAGGAAAGCTATACA ACTGTGGTGAAAGGAAAG[A/G]AAAATCTGCTCTTAAAAGG TTGTGTGCAGACTCACTTGCCCCAGAAACCAGTGCGAAAAC |
| BICF2G6301788 4 | 98 | X | 70145192 | 0.271019 | 0.271019 | GAGATGTGTAAAATTTAATAGAAATGAAACTTGCCAAAACAG ACCTCTGTACTCGTCAGC[A/G]TTCTAAGTCCATCTTTCTG TAGCATGTAAGTAGAATAATGTTCTATTAATTTCCTCTATG |
| BICF2G6305875 98 | 99 | 32 | 39023585 | 0.706931 | 0.293069 | GTTCTTTCTATTCTATCACACATACCACCCCCCTGCCCACAG TACCCCTTTCTGCCATGT[T/C]TCAGACTCCTACACAAGAG GTTCTCTCCTGGCTTCCAGTTAGACAGGCAGGTAAAGCT |
| BICF2P285901 | 100 | 8 | 6743491 | 0.70069 | 0.29931 | TAAAAAAATACAACAGTAGCATTAGAAGACATGCTAAGCGGC TGTATTAGAGAAGGTTAG[T/C]GCTGGCCTGAAGTTTAGAA ACCTTCCCTTCTCTTTTTTTTTTTCCTTCCCTTCTCTTTAA |
| BICF2P811511 | 101 | 32 | 36167454 | 0.30583 | 0.30583 | TCAAGAGTACTAGAGCATCTATAATCAATGGTAAATTGGGGA ACTAGTGAAACAAGTTTA[T/C]AGGACAAATAACATAAATA AGGATTTTTTTTTAAATTTGGAAAATTGTGGAATAATGATA |

EP 2 195 460 B2

| SNP | SEQ ID NO: | Chromosome in canfam 2 | Location in canfam 2 | Allele Frequency | Minor Allele Frequency | SNP Sequence SNP = [wildtype base/alternative base] |
|---|---|---|---|---|---|---|
| BICF2P1146265 | 102 | X | 63433179 | 0.693725 | 0.306275 | AGAATTCAATTTTGGGGAGCCAGGAAACCAGATTAGTTTTCC AAAGGGAAGTGCCATTTG[T/C]ATCTATCCCGGTGGGGCTG CCAAGAATTCCCTGGGGTGGGAGACGGCGCTTCTGTGGATT |
| BICF2P243607 | 103 | X | 57821508 | 0.690523 | 0.309477 | CACCAGAGAGCCCCGCAAGATCATACTGCACAAGGGCTCCAC TGGCCTGGGCTTCAACAT[T/C]GTAGGAGGAGAGGATGGAG AAGGCATTTTGTTTCCTTCATCCTGGCAGGAGGCCCAGCT |
| BICF2P382932 | 104 | X | 64010327 | 0.690335 | 0.309665 | TGGTGATGATTTATCCCCATGTTCAAGATTTATCCTCCCTG TCTCAAGAAATCATGTCA[T/C]TACAGGCATCCTTAAAGTC ACAAGACTGGGAAGTAAATACTGATGAGGTCCAAGACCTGG |
| BICF2P1061734 | 105 | X | 57654632 | 0.69003 | 0.30997 | AGCATAGTGTACCCACATATAAGGTCACATCTGAGGCCAGGG AGTCGGGGTCTTGAAGAT[T/G]ATGACTGATCATGTGCTTG AGGATGATGATGATCATGTGCTTTTCCTGGCTGTGCAGTTG |
| BICF2S2293723 5 | 106 | X | 57492668 | 0.310158 | 0.310158 | gtgtgtgtgtgtgtgtgtgtgtgtTTAATTCTTTGTGAGAAG CCCCTCATTTTGACCTAA[A/G]TTTGGTAGAGGCCCCAGGG GATCTGAGAGGAGAACAAAAGGATAAACCATTTGCTGTTCA |
| BICF2S2293748 9 | 107 | X | 73723672 | 0.687068 | 0.312932 | CCAACTTTCACTAGCATCACAGCCCCTATCAATCTCTGTTCT TTTTTCTGTCAGTACCAT[A/G]TTTGCTCCTACTACATCYA ATCTGTGAGCTCACAGGATGAGGACCAACAGCTGCCCTGAG |
| BICF2P903726 | 108 | 32 | 40883681 | 0.329389 | 0.329389 | TGTCTTACCTCTCTCTATTCCCTTGTCCATAGTAGTATTAAA TATATCTTCCTGAACACA[A/G]ATCTGATCCAGTCTCTTTT TGTAATTAAAAGCCTTTGCTAGCTTTGGTGATCACCTCCAG |
| BICF2P1324008 | 109 | 32 | 40043909 | 0.664179 | 0.335821 | GACCTGACAGATTATGTAGACTTTGTTTTCAAAGGGAGCACC TGCTGGATATACAACATG[A/G]CACTAAATTGTGCTCCACA TCCTTGGCAGAGGTGGGGGGCGGGGCACAAAGGAAGAAACC |

EP 2 195 460 B2

| SNP | SEQ ID NO: | Chromosome in canfam 2 | Location in canfam 2 | Allele Frequency | Minor Allele Frequency | SNP Sequence SNP = [wildtype base/alternative base] |
|---|---|---|---|---|---|---|
| BICF2P320425 | 110 | 8 | 7105593 | 0.336283 | 0.336283 | CAGAGGAAAAGGAGAAGGTCCCACTTAGGGGACTGGAGAGGA GTGGGGGAACATCACCAG[A/C]GCCTTCCTGAGCCAGGCCC CCTGTGGGGAGAAGCTCTCCCCAGGACTGGGTGCCTTTGAA |
| BICF2S23210713 | 111 | 8 | 6397309 | 0.634615 | 0.365385 | tcctccctctccccatccccattctcatgcaagtgtgctctc tctctAAAACACCCCCCC[A/C]CACACACACACACAGACAC AACCAAAtttgggtctcaatgtcttgaccaaggaaaaggca |
| BICF2G63018424 | 112 | X | 66756995 | 0.367793 | 0.367793 | gagaagaaggaggagaaagaggaaaagTATATTTGATGGAAT GAAAAACAAGAGTTCAAT[T/C]TCACTCTGGTCTGGGGTGA CCACTATTAGTCCTTCAACATCTTCCTTGAAGGAATTTTAA |
| BICF2G63015897 | 113 | X | 74179959 | 0.631164 | 0.368836 | CTGGAATTCTGTCAGATCAACATTCAGAGCTCCATCAAATCT GAGGGAAGCAGTGATAGA[A/G]GATACAATTTGACCTTTCA GTCTATTCAGGTTCATGTAGGTTAGGCATTCAATATCAAAG |
| BICF2P305287 | 114 | 8 | 3258209 | 0.371175 | 0.371175 | CCACATGTGGTTACACCACTGTGTTATCCTTCCACCTGTCCC ATCAACCCACCCGCACAT[A/G]TCACAGTGCCTCTGTCCTC AAAGAACACTGTATCCAACACCTCCACATCCTCTCAGCATG |
| BICF2G63016662 | 115 | X | 72647220 | 0.615878 | 0.384122 | ATTCCTATGGTGGGCGCTGCACATTTCCTCCCAGGGGAAGGG CAAGGGTCCTGCATTTCT[A/G]TGCTTTCCAGGGCCTCCGC ACCAAGAGCAATTGCTAGGTCACGCATGCCCCTGCACTTCC |
| BICF2G63017854 | 116 | X | 70302610 | 0.606541 | 0.393459 | CATGTCATCACTAACTAATTTATTAACAAGAGTTTTATTCTT TGAAAAACAAAATCACTC[A/G]CATTACTCAGTTGCTTATT CCTTGATTCATATACAAATGACTGATAACATGAGATAAAAA |
| BICF2P170917 | 117 | 32 | 38039478 | 0.600592 | 0.399408 | GATGATTTAGTTGTTTGAATGATCTGGCATATAAATCTTCCA AATCTGTGTCCATTGGAT[T/C]GCTTACAGTTTAATCTTTT TATTTCTTCCCAGAATCACATTTTTTCATTATTTATCTTTG |

(continued)

| SNP | SEQ ID NO: | Chromosome in canfam 2 | Location in canfam 2 | Allele Frequency | Minor Allele Frequency | SNP Sequence<br>SNP = [wildtype base/alternative base] |
|---|---|---|---|---|---|---|
| BICF2G630588207 | 118 | 32 | 38333881 | 0.425201 | 0.425201 | AGTTAAATTCTGTGAATAACTAGAATCCGTTATACTTTTTCT GAAATGAAGTCTGTAGGC[A/T]TTTCAACAGCAAAAGGAAT TCTGWTTTTYAAAACTATACATAATGCTTCTTAAAAGCCCT |
| BICF2P702899 | 119 | 32 | 39207136 | 0.428854 | 0.428854 | AATGCCAACTTTAAAAACGCATTCAAGGTTTTCCTCTGTAAA TGCATTCCTCATTTTGGA[T/C]GTGATGTAAAATCTTATTC AGTGTTTTGTTTTTTTTTCCCCCCACAGGTCTCAACAATTA |
| BICF2P1388432 | 120 | 8 | 7178740 | 0.446203 | 0.446203 | GGTGGGACCGGCCATCAGCAGGCGGGCCAGCGCCCCACAGAT GTTGTCACGGACCCGATC[A/G]TGGCGCTCCCGTGCCAGGA GGGGCAACAGAAGCCCCAGCAGCTTGGGGAAGTATCTGGTT |
| BICF2P588571 | 121 | 32 | 37214320 | 0.454635 | 0.454635 | AGGGGACTTGTGCTAATCACTGGGCAAATTTTATGAACTTCT GAATTTTAAAGCAAAAGA[A/G]AAGGTGAAAGAATGGAAAG AAGGTGTGAGTGTTTGAGGAAAACTTCTTCTTTGGGGTTGA |
| BICF2S22912518 | 122 | 8 | 6934693 | 0.544872 | 0.455128 | TACACAAGCAAGGCAGTATGCCCTGTCTCCTTCCCTTGGGCC ACCTGCACTTAGACATGG[T/C]AGGTTCCAGTGATGTGTCT AGTCTCTAGCAAGCAGGGCTTGCTTCTGCTCTATCCATCCA |
| BICF2P223099 | 123 | 8 | 7427438 | 0.530602 | 0.469398 | CTGTCCTTGGTCTGGACCTGCTGTGAAGACCAAGTGCTTCCT GAGATCTCTCTGAGTCTA[A/G]TTTCCAGAGCAGTGAGTGA GAAATGAAATGAGCCGAGGATTGCCCTCCCTCCTATGGACT |
| BICF2P568891 | 124 | 8 | 7938712 | 0.475321 | 0.475321 | TAAGCCATCAGCATGGGCTCCTAGGGGTCTGTTCAACTCCCT TGTGGTGTCTTACTGCTC[A/G]AGCAAAGGAACAGTCTGGT ACAGTGGGAGCAAGAGCTGAGGTTGGAGAGTGGGGACACAG |
| BICF2G63019507 | 125 | X | 60714796 | 0.521308 | 0.478692 | GAGGAGGTGGAAGTGATTAAGTTTAAAATTTCTGGGGTGGTT TCTGGCGACATGAAGCTG[A/C]GAGCTAGAATGCCTTTCAA TCTCATAATTTCTTTAATTTGGTGATTATACCAGAGCCACA |

EP 2 195 460 B2

32

| SNP | SEQ ID NO: | Chromosome in canfam 2 | Location in canfam 2 | Allele Frequency | Minor Allele Frequency | SNP Sequence SNP = [wildtype base/alternative base] |
|---|---|---|---|---|---|---|
| BICF2P814468 | 126 | 32 | 37551101 | 0.517787 | 0.482213 | CCTGACAAACACTACCTCTGCTCTTCAAAAGCAATAAGCATT TATTCTGTGACACATTTA[A/G]ATACAAAGTCAATTACAAT AGAGTATAAGTACAATACTAGGGAAAGTACAAAGTCATAYG |
| BICF2P948321 | 127 | 32 | 37526448 | 0.511834 | 0.488166 | GCATGATGAAATCAGAAAAAGTATGTAAGTTTCTAGAAGAAG CTAGATATATGGTAACTT[A/T]GGTCAAATAGAACCATGTA GTGAAAAGAATATGAGTTTTCAAGTTCAATAAAAAACAAAA |
| BICF2P807378 | 128 | 32 | 37648000 | 0.510848 | 0.489152 | ATGCATAAGTTTCCAAAAGAGTTCAGGATTCCAAAATAAAAG CTTCACTAAAAGATTCAT[A/C]GCAAAAGAGTAATGAACAA TTAAAGTCATAGGATATCTAAAATGAAAAACTGTTAGACTG |
| BICF2P175415 | 129 | 8 | 6494289 | 0.489645 | 0.489645 | AGATGGCTTAGTTGTTTCTCTTTCCTCCTGAAGTCCACAGCT TAGTTACTTGGACTCTCC[A/G]AAATaggatcgttggacat ttgaggaaagctctagcatgaaagccatagactaaaaaaca |

### Example 2

*SUMMARY*

The aim of the study was to investigate whether dietary management is effective to influence hepatic copper concentrations in Labradors after treatment with penicillamine, and whether additional treatment with zinc is useful.

The study was conducted on a group of 24 dogs consisting of 12 female and 12 male pure-bred Labradors. The dogs were family members of former patients with copper-associated chronic hepatitis. At the start of the diet trial dogs were clinically healthy but hepatic copper concentrations of 20 dogs were above the reference range of 400 mg/kg dry weight (mean: 894, range: 70-2810 mg/kg dry weight). These concentrations were measured after completion of treatment with penicillamine.

All dogs were fed the same diet. Additional treatment consisted of zinc gluconate (7.5mg/kg PO BID, group 1) or a placebo (group 2). The pharmacist was the only person aware of group allocations until completion of the study. Hepatic copper concentrations and histopathology were assessed along with clinical examinations and blood-work before and after treatment for a mean of 8 months (range 5-13), and 16 months (range 12-25). Plasma zinc concentrations were measured at additional time points during treatment.

Twenty-one Labrador dogs completed the study. At the start of the study the mean age of dogs in group 1 was 4.1 years (range 2.7-8.3). Six dogs were females (5 spayed, 1 intact), and six were male (2 neutered, 4 intact). The mean age of dogs in group 2 was 4.8 years (range 3.6-11.2). Six dogs were female (4 spayed, 2 intact), and 6 dogs were male (2 neutered, 4 intact). Vomiting, anorexia, and diarrhea were observed as adverse effects in 3 dogs of the zinc gluconate supplemented group.

There was a significant difference of hepatic copper concentrations in both groups with dietary management over time (8 months: group 1 p<0.001, group 2 p= 0.001, and 16 months: group1 p= 0.03, group 2 p= 0.04). However, there was no difference in hepatic copper concentrations between groups, prior to treatment (p= 0.65), at recheck-1 (p = 0.52), and at recheck-2 (p = 0.79), suggesting that there is no benefit of further zinc supplementation on hepatic copper accumulation.

The results of this study show that dietary management can be effective to decrease hepatic copper concentrations in Labradors. Adjunctive treatment with zinc did not amplify the de-coppering effect.

The study will now be described in more detail.

*MATERIALS AND METHODS*

*Labrador Retrievers*

The study population consisted of 24 pure-bred Labrador Retrievers that were family members of dogs previously described with CACH. All dogs were registered at the Dutch Labrador Retriever breed club.

All dogs had completed their treatment with penicillamine prior to the start of this study. A medical history was obtained from all dogs and a physical examination was performed. Na-citrated blood samples were taken for analysis of a coagulation profile, including prothrombin time (PT), activated partial thromboplastin time (aPTT) and fibrinogen. Heparin- and EDTA-blood was sampled for analysis of the hepatobiliary enzymes alkaline phosphatase (ALP), alanine aminotransferase (ALT), of bile acids (BA), and for measurement of the platelet count, and plasma zinc concentrations. Liver biopsies were taken according to the Menghini technique given by Rothuizen (Rothuizen J, I. T. (1998) Tijdschr Diergeneeskd 123: 246-252). At least three liver biopsies were taken from each dog. Two biopsies were fixed in 10 percent neutral buffered formalin, and one biopsy was stored in a copper-free container for quantitative copper determination.

All biopsies were histologically assessed. Hepatic tissue was stained with rubeanic acid stain for evaluation of copper distribution and semiquantitation as previously described (Van den Ingh T.S.G.A.M., R. J., Cupery R. (1988) Vet Q 10: 84-89). According to the applied grading system copper scores above 2 are abnormal (0: no copper, 1: solitary liver cells and/or reticulohistiocytic (RHS) cells containing some copper positive granules, 2: small groups of liver cells and/or RHS cells containing small to moderate amounts of copper positive granules, 3: larger groups or areas of liver cells and/or RHS cells containing moderate amounts of copper positive granules, 4: large areas of liver cells and/or RHS cells with many copper positive granules, 5: diffuse presence of liver cells and/or RHS cells with many copper positive granules).

A quantitative assay for copper in liver tissue was performed by neutron activation analysis, according to a protocol described by Teske *et al,* using the facilities described by Bode (Bode, P. (1990) Journal of Trace and Microprobe Techniques 8: 139; Teske et al. (1992) Vet Rec 131: 30-32). Quantitative copper concentrations were measured in lyophilised liver biopsies and reported in mg/kg dry weight liver.

Apart from the copper content, further histological changes were graded on a scale between 0 and 5 in order to allow statistical testing (0 = no histologic signs of inflammation, 1 = reactive hepatitis, 2 = mild chronic hepatitis, 3 = moderate chronic hepatitis, 4 = severe chronic hepatitis, 5 = cirrhosis).

The study was approved by the Utrecht University Institutional Animal Care and Use Committee. Informed owner consent was obtained for all dogs.

*Progression of hepatic copper accumulation without treatment*

In eleven dogs measurement of hepatic copper concentrations was repeated after a mean of 8.7 months (range 6-15 months), prior to any treatment. During this time all animals were fed their usual maintenance diet, which contained dietary copper concentrations between 12-25 mg/kg dry matter and zinc concentrations between 80-270 mg/kg dry matter according to the manufacturers.

*Diet*

All dog owners were provided with the same specially manufactured diet. Approximate analysis of the diet as fed: moisture 57.9%, crude protein 6.1 %, crude fat 5.9%, minerals 1.7%. Metabolizable energy (measured according to Association of American Feed Control Officials protocol): 1650 kcal/kg as fed). The diet contained copper at a concentration of 4.75 mg/kg dry matter, and zinc at a concentration of 102 mg/kg dry matter. The dogs were fed the diet without further dietary supplements and treats. The Labradors of this study received between 420 and 840g diet/day.

*Pharmacokinetic study*

Two unrelated nine-year old, healthy Labrador Retrievers (1 female and I male) were used in a pharmacokinetic study. Food was withheld for a 12-hour period prior to oral administration of zinc, and during the initial 6-hour testing period. Water was freely available. Oral zinc gluconate was administered at a dose of 10 mg/kg in dog 1, and at a dose of 5 mg/kg in dog 2. Heparinized blood samples (4 ml) were collected from the jugular vein before dosing (time 0) and 15, 30, 45, 60, 90, 120 minutes, and 4, 8, 12 and 24 hours after application of the drug. Plasma was stored at -20°C until analysis for zinc using atomic absorption spectrometry.

*Drug preparation, randomization, and blinding*

The choice for zinc-gluconate was made based on our clinical observation that the drug has fewer side effects than the other salts, like acetate or sulphate. The tablets were provided by the Veterinary Pharmacy of the Faculty of Veterinary Medicine, University of Utrecht. Zinc tablets contained 25mg elemental zinc as zinc-gluconate salt (Toppharm Zink 25 gluconaat, Parmalux BV, Uitgeest, NL). Placebo tablets contained 160 mg lactose (Albochin, Pharmachemie BV, Haarlem, NL). Tablets from both groups had an identical appearance.

The Pharmacist randomized group allocations prospectively for sets of six dogs by flipping a coin. Three dogs received the placebo, and three dogs were treated with zinc. On prescription of the clinician, tablets (placebo or zinc) were dispensed according to a randomisation table.

Dosage of the prescription was according to the following scheme:
Bodywelght:

| | | |
|---|---|---|
| <28 kg (<61.6lbs): | 8 tablets BID | (200mg BID) |
| 28 - 35kg (61.6- 77lbs): | 9 tablets BID | (225mg BID) |
| above 35 (>77lbs): | 10 tablets BID | (250mg BID) |

Owners were instructed to give the tablets mixed with small amounts of their diet 30 minutes before feeding. Neither the owner nor the clinician was informed about which treatment each dog received. The randomization table remained in possession of the Pharmacy during the trial and was only revealed upon completion of the trial.

*Statistical analysis*

Pharmacokinetic parameters (WinNonlin 4.0.1. (Pharsight Corporation, 800 West El Camino Real, Mountain View, CA, USA) and statistical analysis (SPSS 11.0 for windows, 2001, SPSS Inc., Chicago, IL, USA) were calculated by use of commercially available software packages. Due to small group sizes a non-parametric statistical test was used for comparison between groups. A Mann-Whitney test was used to detect a difference between hepatic copper concentrations before and after treatment with diet and zinc-gluconate (group-1) at two control examinations (recheck-1 and recheck-2), and after treatment with diet and placebo (group-2) at both control examinations. In addition the test was used to detect a difference between group-1 and group-2 before the study, at recheck-1, and at recheck-2 (significance level p ≤ 0.05).

*RESUL TS*

*Progression of hepatic copper accumulation without treatment*

In eleven Labrador dogs measurement of hepatic copper concentrations was repeated before any treatment was given, and while the dogs were fed their usual maintenance diet. In all but one dog hepatic copper concentrations increased during the time interval of 8.7 months between both measurements from a mean of 1000 mg/kg dry weight (range 290-2370) to a mean of 1626 mg/kg dry weight (range 630-3610). Related to the bodyweight of the patients (mean: 33.9kg, range 25-39.5kg) this was an increase of 18 mg copper per kg bodyweight during 8.7 months. The results are shown in Figure 1.

*Pharmacokinetic study*

Pharmacokinetic parameters calculated from plasma zinc concentrations measured in two dogs after oral application of 10 mg/kg (dog 1) and 5 mg/kg (dog 2) elemental zinc were:

Dog 1 (10 mg/kg): V_F: volume of distribution = 2937.872 ml/kg bodyweight, K01: absorption rate constant = 0.567212 1/hour, K10: elimination rate constant = 0.053728 1/hour, AUC: area under the curve = 63.35272 hr*μg/ml, Cl_F: clearance in relation to the bioavailability = 157.8464 ml/hr/kg, Tmax: time of maximum concentration = 4.589813 hours, Cmax: maximal clearance = 2.659924 μg/ml)

Dog 2 (5 mg/kg): V_F: volume of distribution = 2538.689 ml/kg bodyweight, K01: absorption rate constant = 1.160647 1/hour, K10: elimination rate constant = 0.037886 1/hour, AUC: area under the curve = 51.98513 hr*μg/ml, Cl_F: clearance in relation to the bioavailability = 96.18135 ml/hr/kg, Tmax: time of maximum concentration = 3.047974 hours, Cmax: maximal clearance = 1.754728 μg/ml).

The calculated half-life of zinc was t½= 15.1 hours.
The calculated accumulation rate R was 1.52, from R=1/(1-exp(-k10*24). The dose interval was chosen to be 12 hours.
A dose estimate was 127.0139 ml/hr/kg, calculated from the mean of CL_F from both dogs.
An estimate of the appropriate dosage was based on an intended maximum zinc plasma concentration of 5 μg/ml.

Dosage = Cl_F x intended blood concentration x dose interval = 7.62 mg/kg q 12 hours

*Diet trial and randomized, placebo-controlled zinc application*

At the start of the diet trial hepatic copper concentrations of 20 dogs were above the reference range of 400 mg/kg dry weight (mean: 894, range: 70-2810 mg/kg dry weight). Results from semiquantitative assessment of copper ranged from 0 to 4.5. Histopathological examination of liver biopsies from 17 dogs revealed an elevated hepatic copper content (above 2), which was localized to centrolobular hepatocytes. Staining for copper was normal in 5 dogs, and high normal staining results were obtained from biopsies of 2 dogs with elevated hepatic copper from quantitative analysis. In seven dogs chronic hepatitis was present. CH was characterized by varying degree of hepatocellular apoptosis and necrosis, mononuclear inflammation, regeneration and fibrosis. The activity of the hepatic inflammation was determined by the quantity of inflammation and extent of hepatocellular apoptosis and necrosis. The stage of the disease was determined by the extent and pattern of fibrosis and the presence of cirrhosis Hepatitis was mild in 4 patients, moderate in 2 dogs, and cirrhosis was diagnosed in 1 dog. In thirteen dogs there were no histological signs of inflammation present in the liver, and in biopsies of 4 dogs histopathology revealed reactive changes.
At the start of the study the mean age of dogs in group 1 was 4.1 years (range 2.7-8.3). Six dogs were spayed females, and six were male (2 neutered, 4 intact). The average bodyweight was 33 kg (range 26.2-37.5). The mean hepatic copper concentration of the dogs in group 1 was 961 mg/kg dry weight (range 340-2810). The mean semiquantitative assessment of copper from histological staining was 2-3+ (range 0-4.5).
The mean age of dogs in group 2 was 4.8 years (range 3.6-11.2). Six dogs were female (4 spayed, 2 intact), and 6 dogs were male (2 neutered, 4 intact). The average bodyweight was 32.3 kg (range 25-41.9). The mean hepatic copper concentration of dogs in group 2 was 861 mg/kg dry weight (range 70-1680). There was no difference in hepatic copper concentrations between both groups prior to treatment (p = 0.73). The mean semiquantitative assessment of copper from histological staining was 2-3+ (range 0.5-3).

Three dogs of group-1 did not complete the study. The reason for discontinuation was unrelated to the treatment in all three dogs (one owner felt his dog was getting too old, one owner had personal reasons, and one dog developed a mast cell tumor). Twenty-one dogs completed the study, with at least one control examination (recheck-1). In sixteen dogs liver biopsies were obtained at an additional later time point (recheck-2).

Vomiting, and anorexia were observed adverse effects in 3 dogs of group 1. One of these dogs also had transient small bowel diarrhea. The adverse effects occurred immediately after application of the tablets, and resolved when the tablets were mixed with the diet.

*Blood examinations*

The concentration of bile acids decreased from a mean of 14 $\mu$mol/l (range: 3-101), to a mean of 7.8 (range: 1-39) at recheck-1 and 7.1 (range: 0-21) at recheck-2. The mean concentration of alkaline phosphatase (ALP), and alanine aminotransferase (ALT) remained within the normal range at all examinations. The mean ALP concentration before treatment was 41 U/l (range: 8-111), at recheck-1: mean ALP = 37 U/l (range 12-143), at recheck-2: mean ALP = 37 U/l (range:19-152). The mean ALT concentration before treatment was 28 U/l (range: 10-234), at recheck-1: mean ALT = 47 U/l (range: 11-78), at recheck-2: mean ALT = 51 U/l (26-68).

At the beginning of the study the mean plasma concentrations of zinc were 95 $\mu$g/dl and 96 $\mu$g/dl in group-1 and group-2 respectively. There was no difference in plasma zinc concentrations of either group at recheck-1 and recheck-2 (p values between 0.11 and 0.79). No difference in plasma zinc concentration was found between group 1 and group 2 at any of the three examinations (p values between 0.34 and 0.5). The only time-point at which a difference in plasma zinc concentrations could be found was a blood examination after the initial month of treatment with zinc in group 1. Blood sampling was performed 2-6 hours after zinc application. At this control examination the mean plasma zinc concentration had increased to 165 $\mu$g/dl (range 117-249, p=0.02).

*Hepatic copper measurements*

Quantitative measurement of hepatic copper improved during treatment in both groups. At recheck-1, and at recheck-2, hepatic copper concentrations had decreased significantly in both groups of dogs, compared to the starting point (group-1 at recheck-1: mean 286 mg/kg, range 84-700, p<0.001; group-1 at recheck-2: mean 421 mg/kg, range 220-790, p=0.03, group-2 at recheck-1: mean 277 mg/kg, range 80-450, p= 0.001, group-2 at recheck-2: mean 401 mg/kg, range 118-850, p=0.04). There was no difference in hepatic copper concentrations between both groups at recheck-1 (p=0.52), and there was no difference between groups at recheck-2 (p=0.79). In addition there was no further decrease of hepatic copper concentrations between recheck-1, and recheck-2 (group-1 p=0.44, group-2 p=0.25). The results are shown in Figure 2.

*Histology*

Histological staining for copper improved with treatment. Histological scores for semi-quantitative assessment of copper decreased in group 1 and group 2 at recheck-1 and recheck-2 compared to the starting point (group 1 at recheck-1: p= 0.031, group 1 at recheck-2: p= 0.01, group 2 at recheck-1: p = 0.01, group 2 at recheck-2: p=0.001). There was no difference between both groups at any time-point (p=0.16-0.75).

Histological scoring for severity of inflammation of the liver remained unchanged throughout all examinations of the dogs from both groups (p=0.25-0.45).

*CONCLUSION*

In order to provide patients with CACH with a more balanced long term control of hepatic copper concentrations the aim of this study was to investigate whether dietary management is effective to influence hepatic copper concentrations in Labradors after treatment with penicillamine, and whether additional treatment with zinc is useful. The results of this study show that dietary management can be effective to decrease hepatic copper concentrations. Adjunctive treatment with zinc did not amplify the de-coppering effect.

## Example 3

During an investigation of the effect of zinc on hepatic copper concentration, hepatic copper concentration was measured in 18 pure-bred Labrador Retrievers. Half of the dogs were provided with a supplement of zinc and the other half were provided with a placebo. All of the dogs were fed the diet of Example 2. Hepatic copper concentration was measured using the method of Example 2 at 3 time points: (1) before treatment with penicillamine; (2) after treatment with penicillamine but before treatment with the diet of Example 2; and (3) after the diet treatment. As in Example 2, there was no significant difference between copper levels in dogs treated with zinc and with the placebo (data not shown). However, the use of the diet did have a significant effect on the copper levels. The combined results for dogs treated with zinc and with the placebo are shown in Figure 3 and demonstrate that the low copper diet has a more significant effect on reducing liver copper levels than penicillamine alone.

## Example 4

An example of a foodstuff of the invention is set out below:

| Ingredient (%) | 100 |
|---|---|
| Water | 47.4 |
| Cereals | 28.5 |
| Vegetable by-products | 1.7 |
| Egg and egg by-products | 1.5 |
| Meat and animal by-products | 17 |
| Oil and fat | 2 |
| Minerals and vitamins | 1.9 |

A typical analysis of the amounts of minerals and trace elements in this foodstuff, together with the method used, is provided as follows:

| Analysis | Result (% weight as fed or mg/kg dry matter) | Notes |
|---|---|---|
| Moisture | 63% | Loss by drying at 105 °C for 5 hours |
| Protein | 6.5 % | Protein by Kjeldahl using nitrogen factor 6.25 |
| Fat | 4.2 % | Acid hydrolysis and petroleum ether extraction |
| Ash | 2.2 % | 16hr ramp temperature programme, final ash at 550°C for 5 hr |
| Crude fibre | 1.4 % | EC method OJ L344 26/11/92 P35, Directive 92/89 |
| Copper | 5.4 mg/kg | By flame atomic absorption spectrophotometry |
| Zinc | 229 mg/kg | By flame atomic absorption spectrophotometry |
| Iron | 62 mg/kg | By flame atomic absorption spectrophotometry |
| Calcium | 9 460 mg/kg | By flame atomic absorption spectrophotometry |
| Phosphorus | 5 405 mg/kg | By colorimetric reaction |

## Example 5

A further example of a foodstuff of the invention is as follows:

| Ingredient (%) | 100 |
|---|---|
| Cereals | 39.7 |
| Vegetable by-products | 8.0 |
| Vegetable protein extract | 5.0 |

(continued)

| Ingredient (%) | 100 |
|---|---|
| Meat and animal by-products | 35.6 |
| Oil and fat | 7.5 |
| Minerals and vitamins | 4.2 |

A typical analysis of the amounts of minerals and trace elements in this foodstuff, together with the method used, is provided as follows:

| Analysis | Result (% weight as fed or mg/kg dry matter) | Notes |
|---|---|---|
| Moisture | 8.0 % | Loss by drying at 105 °C for 5 hours |
| Protein | 30.0 % | Protein by Dumas using nitrogen factor 6.25 |
| Fat | 13.0 % | Acid hydrolysis and petroleum ether extraction |
| Ash | 6.3 % | 16hr ramp temperature programme, final ash at 550°C for 5 hr |
| Crude fibre | 4.1 % | EC method OJ L344 26/11/92 P35, Directive 92/89 |
| Copper | 10.9 mg/kg | By flame atomic absorption spectrophotometry |
| Zinc | 245 mg/kg | By flame atomic absorption spectrophotometry |
| Iron | 217 mg/kg | By flame atomic absorption spectrophotometry |
| Calcium | 1 087 mg/kg | By flame atomic absorption spectrophotometry |
| Phosphorus | 760 mg/kg | By colorimetric reaction |

**Claims**

1.  A foodstuff comprising copper at a concentration of at least 4.5 to less than 10 mg/kg dry matter for use in preventing a disease attributable to liver copper accumulation in a dog having genetic inheritance of the Labrador Retriever breed, wherein the dog has no clinical symptoms associated with the liver copper accumulation.

2.  The foodstuff for use in the method according to claim 1, further comprising zinc at a concentration of at least 120 mg/kg dry matter.

3.  The foodstuff for use in the method according to claim 1 or 2, wherein the dog:

    (i) has at least one parent that is a pure-bred Labrador Retriever; and/or
    (ii) has a liver copper concentration of less than 400 mg/kg of dry weight liver; and/or
    (iii) does not have detectable liver disease.

4.  The foodstuff for use in the method according to any one of claims 1 to 3, wherein the method comprises preventing copper-associated chronic hepatitis.

5.  The foodstuff for use in the method according to any one of the preceding claims, wherein the method comprises providing the dog the foodstuff throughout the dog's life.

6.  Use of copper in the manufacture of a foodstuff for a dog having genetic inheritance of the Labrador Retriever breed, wherein the foodstuff comprises copper at a concentration of at least 4.5 to less than 10 mg/kg dry matter and is for use in preventing a disease attributable to liver copper accumulation in said dog, and wherein the dog has no clinical symptoms associated with liver copper accumulation.

**7.** The use according to claim 6, wherein the use of the foodstuff comprises providing the dog the foodstuff throughout the dog's life.

**8.** A pack comprising a foodstuff having copper at a concentration of at least 4.5 to less than 10 mg/kg dry matter and a zinc supplement for providing a concentration of at least 120 mg/kg dry matter for simultaneous, separate or sequential use in preventing a disease attributable to liver copper accumulation in a dog having genetic inheritance of the Labrador Retriever breed, wherein the dog has no clinical symptoms associated with liver copper accumulation.

**9.** The pack for use according to claim 8, wherein the use comprises providing the dog the foodstuff throughout the dog's life.

**10.** A labelled foodstuff for use in the method according to any one of claims 1 to 5 or labelled pack for use according to claim 8 or 9.

**Patentansprüche**

**1.** Nahrungsmittel, welches Kupfer bei einer Konzentration von mindestens 4,5 bis weniger als 10 mg/kg Trockenmasse umfasst, zur Verwendung beim Verhindern einer Erkrankung, die auf Kupferanreicherung in der Leber eines Hunds mit einem genetischen Erbgut der Rasse Labrador Retriever zurückführbar ist, wobei der Hund keine der Kupferanreicherung in der Leber zugeordnete klinische Symptome aufweist.

**2.** Nahrungsmittel zur Verwendung bei dem Verfahren nach Anspruch 1, welches weiterhin Zink bei einer Konzentration von mindestens 120 mg/kg Trockenmasse umfasst.

**3.** Nahrungsmittel zur Verwendung bei dem Verfahren nach Anspruch 1 oder 2, wobei der Hund:

(i) mindestens ein Elternteil hat, das ein reinrassiger Labrador Retriever ist; und/oder
(ii) eine Kupferanreicherung in der Leber von weniger als 400 mg/kg Trockenmasse Leber aufweist; und/oder
(iii) keine nachweisbare Lebererkrankung aufweist.

**4.** Nahrungsmittel zur Verwendung bei dem Verfahren nach einem der Ansprüche 1 bis 3, wobei das Verfahren das Verhindern einer mit Kupfer in Zusammenhang gebrachten chronischen Hepatitis umfasst.

**5.** Nahrungsmittel zur Verwendung bei dem Verfahren nach einem der vorhergehenden Ansprüche, wobei das Verfahren das lebenslange Versorgen des Hunds mit dem Nahrungsmittel umfasst.

**6.** Verwendung von Kupfer bei der Herstellung eines Nahrungsmittels für einen Hund mit dem genetischen Erbgut der Rasse Labrador Retriever, wobei das Nahrungsmittel Kupfer bei einer Konzentration von mindestens 4,5 bis weniger als 10 mg/kg Trockenmasse umfasst und zur Verwendung beim Verhindern einer Erkrankung dient, die auf Kupferanreicherung in der Leber des Hunds zurückführbar ist, und wobei der Hund keine einer Kupferanreicherung in der Leber zugeordnete klinische Symptome aufweist.

**7.** Verwendung nach Anspruch 6, wobei die Verwendung des Nahrungsmittels das lebenslange Versorgen des Hunds mit dem Nahrungsmittel umfasst.

**8.** Packung, welche ein Nahrungsmittel mit Kupfer bei einer Konzentration von mindestens 4,5 bis weniger als 10 mg/kg Trockenmasse und einem Zinkzusatz zum Vorsehen einer Konzentration von mindestens 120 mg/kg Trockenmasse für gleichzeitige, separate oder aufeinanderfolgende Verwendung beim Verhindern einer Erkrankung, die auf Kupferanreicherung in der Leber eines Hunds mit dem genetischen Erbgut der Rasse Labrador Retriever zurückführbar ist, umfasst, wobei der Hund keine einer Kupferanreicherung in der Leber zugeordnete klinische Symptome aufweist.

**9.** Packung zur Verwendung nach Anspruch 8, wobei die Verwendung das lebenslange Versorgen des Hunds mit dem Nahrungsmittel umfasst.

**10.** Etikettiertes Nahrungsmittel zur Verwendung bei dem Verfahren nach einem der Ansprüche 1 bis 5 oder etikettierte Packung zur Verwendung nach Anspruch 8 oder 9.

**Revendications**

1. Aliments comprenant du cuivre à une concentration d'au moins 4,5 à moins de 10 mg/kg de matière sèche à utiliser dans la prévention d'une maladie imputable à une accumulation de cuivre dans le foie chez un chien ayant une hérédité génétique de la race Labrador Retriever, où le chien ne présente pas de symptômes cliniques associés à l'accumulation de cuivre dans le foie.

2. Aliments à utiliser dans le procédé selon la revendication 1, comprenant en outre du zinc à une concentration d'au moins 120 mg/kg de matière sèche.

3. Aliments à utiliser dans le procédé selon la revendication 1 ou 2, où le chien :

    (i) a au moins un parent qui est un Labrador Retriever de pure race ; et/ou
    (ii) a une concentration de cuivre dans le foie inférieure à 400 mg/kg de poids à sec ; et/ou
    (iii) n'a pas de maladie du foie décelable.

4. Aliments à utiliser dans le procédé selon l'une quelconque des revendications 1 ou 3, où le procédé consiste à éviter une hépatite chronique associée au cuivre.

5. Aliments à utiliser dans le procédé selon l'une quelconque des précédentes revendications, où le procédé consiste à fournir au chien les aliments pendant toute la vie du chien.

6. Utilisation de cuivre dans la fabrication d'aliments pour un chien ayant une hérédité génétique de la race Labrador Retriever, dans laquelle les aliments comprennent du cuivre à une concentration d'au moins 4,5 à moins de 10 mg/kg de matière sèche et sont destinés à prévenir une maladie imputable à une accumulation de cuivre dans le foie chez ledit chien, et où le chien ne présente pas de symptômes cliniques associés à une accumulation de cuivre dans le foie.

7. Utilisation selon la revendication 6, dans laquelle l'utilisation des aliments consiste à fournir au chien les aliments pendant toute la vie du chien.

8. Pack comprenant des aliments ayant du cuivre à une concentration d'au moins 4,5 à moins de 10 mg/kg de matière sèche et un supplément de zinc pour fournir une concentration d'au moins 120 mg/kg de matière sèche pour une utilisation simultanée, séparée ou séquentielle dans la prévention d'une maladie imputable à une accumulation de cuivre dans le foie chez un chien ayant une hérédité génétique de la race Labrador Retriever, où le chien ne présente pas de symptômes cliniques associés à une accumulation de cuivre dans le foie.

9. Pack à utiliser selon la revendication 8, dans lequel l'utilisation consiste à fournir au chien les aliments pendant toute la vie du chien.

10. Aliments étiquetés à utiliser dans le procédé selon l'une quelconque des revendications 1 à 5 ou pack étiqueté à utiliser selon la revendication 8 ou 9.

Figure 1

Figure 2

Figure 3

Figure 4

INPUT
20

COMPARISON MODULE
30

DATABASE
10

DETERMINING MEANS
40

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *PCR Methods and Applications,* 1994, vol. 3, 268-71
- *Proc. Natl. Acad. Sci.,* 1998, vol. 85, 4397-4401
- **DEVEREUX et al.** *Nucleic Acids Research,* 1984, vol. 12, 387-395
- **ALTSCHUL S. F.** *J Mol Evol,* 1993, vol. 36, 290-300
- **ALTSCHUL, S, F et al.** *J Mol Biol,* 1990, vol. 215, 403-10
- **HENIKOFF ; HENIKOFF.** *Proc. Natl. Acad. Sci. USA,* 1992, vol. 89, 10915-10919
- **KARLIN ; ALTSCHUL.** *Proc. Natl. Acad Sci. USA,* 1993, vol. 90, 5873-5787
- **MADDOX et al.** *J. Exp. Med.,* 1993, vol. 158, 1211-1226
- **HARLOW ; LANE.** Antibodies: A Laboratory Manual. Cold Spring Harbor Laboratory Press, 1988
- **KOHLER ; MILSTEIN.** *Nature,* 1975, vol. 256, 495-497
- **ROTHUIZEN J, I. T.** *Tijdschr Diergeneeskd,* 1998, vol. 123, 246-252
- **VAN DEN INGH T.S.G.A.M., R. J. ; CUPERY R.** *Vet Q,* 1988, vol. 10, 84-89
- **BODE, P.** *Journal of Trace and Microprobe Techniques,* 1990, vol. 8, 139
- **TESKE et al.** *Vet Rec,* 1992, vol. 131, 30-32